# EUROPEAN PATENT SPECIFICATION

(11) **EP 2 533 634 B1**
(45) Date of publication and mention of the grant of the patent: **21.10.2015**
(21) Application number: 11742896.1
(22) Date of filing: 11.02.2011
(51) Int. Cl.: A61K 31/275, A61K 38/16, A61K 38/21, A61K 31/225, A61P 25/00

(54) **NEUROPROTECTION IN DEMYELINATING DISEASES**
NERVENSCHUTZ BEI ENTMARKUNGSERKRANKUNGEN
NEUROPROTECTION DANS LES MALADIES DÉMYÉLINISANTES

(30) Priority: 12.02.2010 US 304325 P; 06.04.2010 US 321486 P
(43) Date of publication of application: 19.12.2012
(60) Divisional application: 15184650.8
(73) Proprietor: Biogen MA Inc., Cambridge, MA 02142 (US)
(72) Inventor: GOELZ, Susan, Portland, OR 97202 (US); DAWSON, Kate, Westwood, MA 02090 (US); LINKER, Ralf, 44809 Bochum (DE); GOLD, Ralf, 44807 Bochum (DE)
(74) Representative: Pohlman, Sandra M.
(86) International application number: PCT/US2011/024594
(87) International publication number: WO 2011/100589

(56) References cited:
- US-A1- 2008 089 861
- US-A1- 2008 227 847
- US-A1- 2009 104 142
- US-B1- 6 436 992
- US-B2- 6 800 735
- US-B2- 7 320 999
- SCZESNY-KAISER M ET AL: "Synergismus von Interferon [beta]-1b und Fumarsäure?. [Synergism of Interferon beta-1b and Fumaric Acid?", AKTUELLE NEUROLOGIE, THIEME, STUTTGART, DE, vol. 36, no. Supplement 3, 2009, pages S284-S286, XP008162663, ISSN: 0302-4350, DOI: 10.1055/S-0029-1220420 [retrieved on 2009-09-02]

## Description

### FIELD OF THE INVENTION

Provided are methods and compositions for treating demyelinating disorders and related disorders of the nervous system, including for example, multiple sclerosis.

### BACKGROUND OF THE INVENTION

Fumaric acid esters have demonstrated beneficial effects in myelin oligodendrocyte glycoprotein induced experimental autoimmune encephalomyelitis (MOG-EAE) (See e.g., WO 2008/096271A2) as well as on MRI parameters of disease activity in a Phase II trial in relapsing remitting multiple sclerosis. Fumaric acid esters might offer a novel mechanism of action that includes axonal protection via Nrf2-mediated anti-oxidative pathways.

Multiple sclerosis (MS) is an autoimmune disease with the autoimmune activity directed against central nervous system (CNS) antigens. The disease is characterized by discrete inflammation, known as lesions, in the CNS, leading to the loss of the myelin sheathing around neuronal axons (demyelination), axonal loss, and the eventual death of neurons, oligodenrocytes and glial cells. For a comprehensive review of MS and current therapies, see, e.g., McAlpine's Multiple Sclerosis, by Alastair Compston et al., 4th edition, Churchill Livingstone Elsevier, 2006.

An estimated 2,500,000 people in the world suffer from MS. It is one of the most common CNS diseases in young adults. MS is a chronic, progressing, disabling disease, which generally strikes its victims some time after adolescence, with diagnosis generally made between 20 and 40 years of age, although onset may occur earlier. The disease is not directly hereditary, although genetic susceptibility plays a part in its development. MS is a complex disease with heterogeneous clinical, pathological and immunological phenotype.

There are four major clinical types of MS: 1) relapsing-remitting MS (RR-MS), characterized by clearly defined relapses with full recovery or with sequelae and residual deficit upon recovery; periods between disease relapses characterized by a lack of disease progression; 2) secondary progressive MS (SP-MS), characterized by initial relapsing remitting course followed by progression with or without occasional relapses, minor remissions, and plateaus; 3) primary progressive MS (PP-MS), characterized by disease progression from onset with occasional plateaus and temporary minor improvements allowed; and 4) progressive relapsing MS (PR-MS), characterized by progressive disease onset, with clear acute relapses, with or without full recovery; periods between relapses characterized by continuing progression.

Clinically, the illness most often presents as a relapsing-remitting disease and, to a lesser extent, as steady progression of neurological disability. Relapsing-remitting MS (RR-MS) presents in the form of recurrent attacks of focal or multifocal neurologic dysfunction. Attacks may occur, remit, and recur, seemingly randomly over many years. Remission is often incomplete and as one attack follows another, a stepwise downward progression ensues with increasing permanent neurological deficit. The usual course of RR-MS is characterized by repeated relapses associated, for the majority of patients, with the eventual onset of disease progression. The subsequent course of the disease is unpredictable, although most patients with a relapsing-remitting disease will eventually develop secondary progressive disease. In the relapsing-remitting phase, relapses alternate with periods of clinical inactivity and may or may not be marked by sequelae depending on the presence of neurological deficits between episodes. Periods between relapses during the relapsing-remitting phase are clinically stable. On the other hand, patients with progressive MS exhibit a steady increase in deficits, as defined above and either from onset or after a period of episodes, but this designation does not preclude the further occurrence of new relapses.

MS pathology is, in part, reflected by the formation of focal inflammatory demyelinating lesions in the white matter, which are the hallmarks in patients with acute and relapsing disease. In patients with progressive disease, the brain is affected in a more global sense, with diffuse but widespread (mainly axonal) damage in the normal appearing white matter and massive demyelination also in the grey matter, particularly, in the cortex.

Most current therapies for MS are aimed at the reduction of inflammation and suppression or modulation of the immune system. A number of clinical trials have shown that the suppression of inflammation in chronic MS rarely significantly limits the accumulation of disability through sustained disease progression, suggesting that neuronal damage and inflammation are independent pathologies. Thus, in MS, neurodegeneration appears to progress even in the absence of significant inflammation. Therefore, slowing demyelination, or promoting CNS remyelination as a repair mechanism, or otherwise preventing axonal loss and neuronal death are some of the important goals for the treatment of MS, especially, in the case of progressive forms of MS such as SP-MS.

There are currently several treatments approved for relapsing-remitting MS (RRMS). Some of these treatments include interferon products (e.g., Avonex^{®}, Betaseron^{®}, and Rebif^{®}), and glatiramer acetate [GA] (Copaxone^{®}). Interferons and GA each provide a modest, but important, clinical benefit; they each have demonstrated a mean reduction in relapse rate of approximately 29% to 33% over 2 years (IFN-beta Multiple Sclerosis Study Group [No authors listed] Interferon beta-1b is effective in relapsing-remitting multiple sclerosis. I. Clinical results of a multicenter, randomized, double-blind, placebocontrolled trial. The IFN-beta Multiple Sclerosis Study Group. Neurolog. 1993;43(4):655-61; PRISMS. Randomised double-blind placebo-controlled study of interferon β-1a in relapsing/remitting multiple sclerosis. PRISMS (Prevention of Relapses and Disability by Interferon β-1a Subcutaneously in Multiple Sclerosis) Study Group. Lancet. 1998;352:1498-504; Jacobs L, Cookfair D, Rudick R, et al. Intramuscular interferon β-1a for disease progression in relapsing multiple sclerosis. The Multiple Sclerosis Collaborative Research Group (MSCRG). Ann Neurol. 1996;39:285-94; Johnson KP, Brooks BR, et al. Copolymer 1 reduces relapse rate and improves disability in relapsing-remitting multiple sclerosis: results of phase III multicentre, double-blind, placebo controlled trial. Neurology 1995; 45:1268-1276). Although interferons and GA have acceptable efficacy profiles, they also possess features that reduce patient compliance. These approved therapies for MS require frequent injections and often cause side effects that limit compliance and lead to discontinuation. Furthermore, patients who continue to have disease activity while on monotherapy with one of these treatments would benefit from combination therapies. Therapies that can be safely combined with approved treatments for MS are needed to improve compliance and overall efficacy. Combination therapies may also provide an alternative to therapies with higher risk profiles, such as natalizumab.

Fumaric acid esters, such as dimethyl fumarate (DMF), have been previously proposed for the treatment of MS (see, e.g., Schimrigk et al., Eur. J. Neurol., 2006, 13(6):604-10; Drugs R&D, 2005, 6(4):229-30; U.S. Patent No. 6,436,992). Combinations of an NMDA receptor antagonist such as memantine, rimantadine and amantadine with a fumarate agent, such as DM F have also previously been proposed for the treatment of MS (see, e.g., US Publication No. 2008/0089861). DMF can. exert neuroprotective effects such as reduction in demyelination and axonal damage in a mouse MS model with characteristic features of advanced stages of chronic forms of MS.

### BRIEF SUMMARY OF THE INVENTION

Given the good safety profile of fumaric acid esters, combination therapies with fumaric acid esters, e.g., DMF, in combination with glatiramer acetate (or related copolymers) or interferon-beta for the treatment of neurological disorders, such as MS are warranted.

### Method I:

An embodiment of the invention is a combination therapy consisting of (a) dimethylfumarate (DMF) and/or monomethyl fumarate (MMF), and (b)glatiramer acetate for use in treating a subject having multiple sclerosis (MS).

Described herein is a medical use for treating a subject having a neurological disorder (e.g., MS), wherein the use includes administering to the subject: (a) a therapeutically effective amount of at least one compound of Formula I: 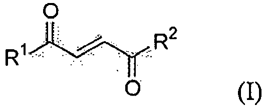 in which R¹ and R² are independently selected from OH, O⁻, and (C₁₋₆)alkoxy, or a pharmaceutically acceptable salt thereof; and (b) a therapeutically effective amount of glatiramer acetate. The neurological disorder is characterized by demyelination and/or axonal loss. In the invention, the neurological disorder is MS.

As described herein, the compound of Formula I is selected from monoalkyl fumarates (i.e., at least one of R¹ and R² is alkoxy), dialkyl fumarates (i.e., R¹ and R² are both alkoxy), and combinations thereof. In some embodiments according to the invention, the at least one compound of Formula I is chosen from dimethyl fumarate (DMF), monomethyl fumarate (MMF), and combinations thereof. In some embodiments according to the invention, the at least one compound of Formula I is chosen from dimethyl fumarate (DMF) and monomethyl fumarate (MMF). In some embodiments according to the invention, the at least one compound of Formula I is DMF. In some embodiments according to the invention, the at least one compound of Formula I is MMF. In some embodiments according to the invention, the at least one compound of Formula I is a combination of DMF and MMF. Additional compounds of Formula I useful in any of the above uses are described in U.S. Patent 6,509,376 to Joshi et al.

In some embodiments according to the invention DMF and/or MMF and glatiramer acetate are administered in amounts and for periods of time sufficient to reduce demyelination and/or axonal death in the subject. In some embodiments according to the invention DMF and/or MMF and glatiramer acetate are administered in amounts and for periods of time sufficient to reduce the relapse rate in the subject.

In some embodiments according to the invention DMF and/or MMF and the glatiramer acetate are present in a single pharmaceutical formulation.

In some embodiments according to the invention 20 mg per day of glatiramer acetate is administered to the subject. In some embodiments according to the invention, less than 20 mg per day of glatiramer acetate is administered to the subject. Other useful dosages for glatiramer acetate in the medical uses of the invention are described herein.

### Method II:

A further embodiment of the invention provides a combination therapy consisting of (a) DMF and/or MMF, and (b) interferon-beta, for use treating a subject having multiple sclerosis (MS). Also described are medical uses for treating a subject having a neurological disorder (e.g., MS), wherein the use includes administering to the subject: (a) a therapeutically effective amount of at least one compound of Formula I: 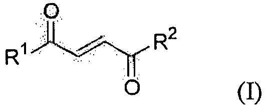 in which R¹ and R² are independently selected from OH, O⁻, and (C₁₋₆)alkoxy, or a pharmaceutically acceptable salt thereof; and (b) a therapeutically effective amount of interferon-beta. The neurological disorder is characterized by at least one of demyelination and axonal loss. In the invention, the neurological disorder is MS.

As described herein, the compound of Formula I is selected from monoalkyl fumarates (i.e., at least one of R¹ and R² is alkoxy), dialkyl fumarates (i.e., R¹ and R² are both alkoxy), and combinations thereof. In some embodiments according to the invention, the at least one compound of Formula I is chosen from dimethyl fumarate (DMF), monomethyl fumarate (MMF), and combinations thereof. In some embodiments according to the invention, the at least one compound of Formula I is chosen from dimethyl fumarate (DMF) and monomethyl fumarate (MMF). In some embodiments according to the invention,
the at least one compound of Formula I is DMF. In some embodiments according to the invention, the at least one compound of Formula I is MMF. In some embodiments according to the invention, the at least one compound of Formula I is a combination of DMF and MMF. Additional compounds of Formula I useful in the above uses are described in U.S. Patent 6,509,376 to Joshi et al.

In some embodiments according to the invention DMF and/or MMF and interferon-beta are administered in amounts and for periods of time sufficient to reduce demyelination and/or axonal death in the subject. In some embodiments according to the invention DMF and/or MMF and interferon-beta are administered in amounts and for periods of time sufficient to reduce accumulation of disability in the subject. In some embodiments according to the invention DMF and/or MMF and interferon-beta are administered in amounts and for periods of time sufficient to reduce the relapse rate in the subject.

In some embodiments of the invention, the interferon-beta is selected from interferon-beta 1a and interferon-beta 1b. In some embodiments according to the invention, the interferon-beta is interferon-beta 1a. In some embodiments according to the invention, the interferon-beta 1a is selected from AVONEX® and Rebif®. In some embodiments according to the invention, the interferon-beta is interferon-beta 1b. In some embodiments according to the invention, the Interferon-beta 1b is selected from Betaseron®, Extavia® and Ziferon®. Other interferon-beta useful in any of the uses of the invention are provided herein.

The interferon-beta (e.g., AVONEX®) is administered at a dose of 30 mcg once a week. In some embodiments according to the invention, the interferon-beta (e.g., AVONEX®) is administered at a dose of 30 mcg injected intramuscularly (IM) once a week. In some embodiments the interferon-beta (e.g., AVONEX®) is administered at a dose of less than 30 mcg once a week. In some embodiments the interferon-beta (e.g., AVONEX®) is administered at a dose of less than 30 mcg injected intramuscularly once a week. Other useful dosages for interferon-beta (e.g., AVONEX®) in the methods of the invention are described herein.

In some embodiments according to Method I and Method II, DMF is administered to the subject in certain amounts: In some embodiments according to the invention, 120 mg of DMF is administered to the subject at one time. In some embodiments 240 mg of DMF is administered to the subject at one time. In some embodiments according to the invention 120 mg of the DMF is administered to the subject three times per day (TID) equivalent to a total daily dose of 360 mg per day. In some embodiments according to the invention 120 mg of the DMF is administered to the subject two times per day (BID) equivalent to a total daily dose of 240 mg per day. In some embodiments according to the invention, 240 mg of the DMF is administered to the subject three times per day (TID) equivalent to a total daily dose of 720 mg per day. In some embodiments according to the invention, 240 mg of the DMF is administered to the subject two times per day (BID) equivalent to a total daily dose of 480 mg per day.

In some embodiments according to the invention, the DMF is provided to the subject in a unit dosage form comprising 120 mg of DMF. In some embodiments according to the invention a unit dosage form comprising 120 mg DMF is administered to the subject once per day. In some embodiments according to the invention, a unit dosage form comprising 120 mg DMF is administered to the subject twice per day, equivalent to a total daily dose of 240 mg of DMF per day. In some embodiments according to the invention, a unit dosage form comprising 120 mg DMF is administered to the subject three times per day, equivalent to a total daily dose of 360 mg per day.

In some embodiments according to the invention, the amount of DMF administered to the subject is between 50 mg and 2000 mg per day. In some embodiments according to the invention, the amount of DMF administered to a human subject is between 100 mg and 1000 mg per day. In some embodiments according to the invention, the amount of DMF administered to a human subject is between 100 mg and 800 mg per day.

In some embodiments according to the invention, the DMF is administered at least about one hour before or at least about one hour after food is consumed by the subject.

As described herein according to Method I and Method II, the neurological disorder is selected from multiple sclerosis (MS), Huntington's disease, Alzheimer's disease, Parkinson's disease, optic neuritis, Devic disease, transverse myelitis, acute disseminated encephalomyelitis, adrenoleukodystrophy and adrenomyeloneuropathy, acute inflammatory demyelinating polyneuropathy (AIDP), chronic inflammatory demyelinating polyneuropathy (CIDP), acute transverse myelitis, progressive multifocal leucoencephalopathy (PML), acute disseminated encephalomyelitis (ADEM) and other hereditary disorders, such as leukodystrophies, Leber's optic atrophy, and Charcot-Marie-Tooth disease. In some cases, the neurological disorder is an auto-immune disease.

As described herein according to Method I and Method II, the neurological disorder is selected from multiple sclerosis (MS), Huntington's disease, Parkinson's disease, and Alzheimer's disease. In the embodiments according to any of the above embodiments of Method I and Method II, the neurological disorder is MS. In some embodiments according to any of the above methods, the MS is selected from relapsing remitting MS, secondary progressive MS, primary progressive MS, progressive relapsing MS, and clinically isolated syndrome (CIS).

As described herein, the subject has a progressive form of a demyelinating disorder. In some embodiments according to the invention, the subject has a progressive form of MS. In some embodiments according to the invention, the subject exhibits at least a 1-point increase on the Enhanced Disability Status Scale (EDSS) over a period of about one year prior to the initiation of treatment with the compound DMF and/or MMF and glatiramer acetate or treatment with the compound DMF and/or MMF and interferon-beta. In some embodiments according to the invention, the subject exhibits at least a 25% increase in T1 lesion load over a period of about one year prior to the initiation of treatment with the compound DMF and/or MMF and glatiramer acetate or treatment with the compound of DMF and/or MMF and interferon-beta. In some embodiments according to the invention, the subject has an EDSS score of at least 3. In some embodiments according to the invention, the subject has more than 10 hypointense T1 lesions. In some embodiments according to the invention, the subject is a human patient.

Other features and embodiments will be apparent from the following description and the claims.

### BRIEF DESCRIPTION OF THE FIGURES

Figure 1 is a graphical representation showing the mean clinical score in a mouse MOG-EAE model for mice treated with DMF at 15 mg/kg twice daily via oral gavage (Example 1).
Figure 2 contains pictures of spinal cord tissues showing demyelination, relative axonal density and gliosis in a mouse MOG -EAE model (Example 1).
Figure 3 , is a graphical representation comparing the mean clinical score in a mouse MOG-EAE model for mice co-injected with glatiramer acetate and MOG antigen. A control group received MOG alone, while two experimental groups received doses of 100 or 500 mcg of glatiramer acetate (Example 2).
Figure 4 is a graphical representation showing the results of a DMF/GA co-therapy during chronic MOG-EAE (Example 3).
Figure 5 is a graphical representation showing the results of a DMF/GA co-therapy during chronic MOG-EAE and represents pooled data from two experiments (n= 12 per group) (Example 3).
Figure 6 is a representation showing the results of DMF/GA co-therapy during chronic MOG-EAE and a histological analysis of mice spinal cords for various treatment groups during the chronic phase (Example 3).
Figure 7 is a representation showing the results of DMF/GA co-therapy during chronic MOG-EAE with respect to T cell infiltration (Example 3).
Figure 8 is a representation showing the results of DMF/GA co-therapy during chronic MOG-EAE with respect to macrophage infiltration (Example 3).
Figure 9 is a representation showing the results of DMF/GA co-therapy during chronic MOG-EAE with respect to axonal loss and destruction (Example 3).
Figure 10 is a representation comparing the mean clinical score in a mouse MOG-EAE model for mice subjected to a DMF/GA co-therapy during the short term course of MOG-EAE (Examples 3 and 8). DMF/GA co-therapy leads to sustained amelioration of the clinical disease course. Figure 10A represents a single experiment, while Figure 10B depicts a pool of three different experiments.
Figure 11 is a representation comparing the mean clinical score in a mouse MOG-EAE model for mice subjected to a DMF/IFN-beta co-therapy (Example 4).
Figure 12 is a representation showing the results of DMF/GA co-therapy during chronic MOG-EAE with respect to axonal loss and destruction (axonal densities). DMF/GA co-therapy leads to a preservation of axons on day 26 p.i. (Example 8).
Figure 13 is a representation showing the results of DMF/GA co-therapy during chronic MOG-EAE with respect to macrophage infiltration. DMF as well as DMF/GA co-therapy lead to a reduction of macrophage/microglia in EAE lesions on day 26 p.i. (Example 8).
Figure 14 is a representation showing the results of DMF/GA co-therapy during chronic MOG-EAE with respect to T cell infiltration. DMF/GA co-therapy leads to a reduction ofT-cells in EAE lesions at day 26 p.i. (Example 8).
Figure 15 is a representation comparing the mean clinical score in a mouse MOG-EAE model for mice subjected to a DMF/IFN-beta co-therapy. DMF/IFN-beta co-therapy leads to a sustained amelioration of the clinical disease course (Example 8).
Figure 16 is a representation showing the results of DMF/IFN-beta co-therapy during chronic MOG-EAE with respect to inflammatory index after hematoxylin and eosin (HE) staining. DMF/IFN-beta co-therapy reduces inflammatory foci in the spinal cord on day 18 of MOG-EAE (Example 8).
Figure 17 is a representation showing the results of DMF/IFN-beta co-therapy during chronic MOG-EAE with respect to T cell infiltration. DMF/IFN-beta co-therapy does not lead to a significant reduction of T-cells in established lesions at day 18 p.i. (Example 8).

### DETAILED DESCRIPTION OF THE INVENTION

### Definitions

Certain terms are defined in this section; additional definitions are provided throughout the description.
Mcg = microgram

"Dimethyl fumarate" or "DMF" refers to the compound having the structure

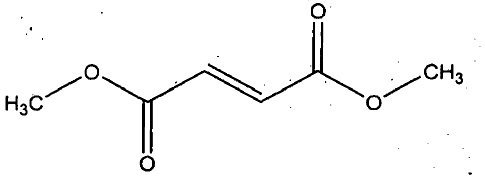

"Methyl hydrogen fumarate" or "MMF" refers to the compound having the structure 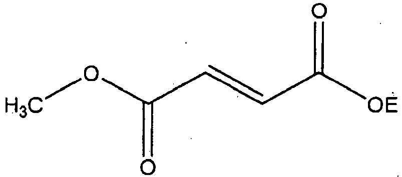 or a pharmaceutically acceptable salt thereof, wherein E is H or a negative charge. MMF may also be referred to as (E)-4-methoxy-4-oxobut-2-enoic acid.

The term "(C₁₋₆)alkoxy" is used in its generally accepted meaning. For example, the term "(C₁₋₆)alkoxy" means an alkoxy group having the formula, -OR^{a}, wherein R^{a} is a straight or branched alkyl radical having from 1 to 6 carbon atoms. Exemplary (C₁₋₆)alkoxy groups include methoxy, ethoxy, propyloxy, *iso*-propyloxy, n-butyloxy, *iso-*butyloxy, sec-butyloxy, and *n*-pentyloxy. In some embodiments, the (C₁₋₆)alkoxy is methoxy.

"Interferon-beta" or "IFN-beta" includes any interferon-beta protein (e.g., any naturally occurring interferon-beta protein), whether isolated from a tissue or blood or obtained by a recombinant technique. In some embodiments, the interferon-beta is human interferon-beta. In some embodiments, the human interferon beta is recombinantly produced in mammalian cells. In some embodiments, the human interferon-beta is recombinantly produced in bacterial cells, such as *e. coli.* In some embodiments the interferon-beta is interferon-beta 1a. In some embodiments, the interferon beta 1a is recombinantly produced in mammalian cells. In some embodiments, the interferon beta 1a is recombinantly produced in bacterial cells. In some embodiments, the interferon-beta 1a is selected from Rebif®, Avonex® and Cinno Vex®, which are commercially available forms of interferon-beta 1a (e.g., Avonex® and Rebif® are marketed in the United States) but other forms are also encompassed. In some embodiments, the interferon-beta 1a is a biosimilar or biogeneric form of Avonex® or Rebif®.

In some embodiments, the amino acid sequence of the interferon-beta 1a is at least 50% identical, at least 60% identical, at least 70% identical, at least 80% identical or at least 90% identical to the amino acid sequence of natural human interferon-beta 1a. In some embodiments, the amino acid sequence of the interferon-beta 1a is essentially identical to the amino acid sequence of natural human interferon-beta 1a.

In some embodiments the interferon-beta is interferon-beta 1b. In some embodiments, the interferon-beta 1b is recombinantly produced in bacterial cells (e.g., modified *e. coli*). In some embodiments, the interferon-beta 1b is recombinantly produced in mammalian cells. In some embodiments, the amino acid sequence of the interferon-beta 1b is at least 50% identical, at least 60% identical, at least 70% identical, at least 80% identical or at least 90% identical to the amino acid sequence of natural human interferon-beta 1b. In some embodiments, the amino acid sequence of the interferon-beta 1b is essentially identical to the amino acid sequence of natural human interferon-beta 1b.

In some embodiments, the interferon-beta 1b is selected from Betaseron® (also referred to as Betaferon®), Extavia® and ZIFERON®, which are commercially available forms of interferon-beta 1b (e.g., Betaseron® is marketed in the United States), but other forms are also encompassed. In some embodiments, the interferon-beta 1b is a biosimilar or biogeneric form of Betaseron®, Extavia® or ZIFERON®.

Modified forms of interferon-beta are also encompassed by the term interferon-beta. For example, an interferon-beta can be modified by deleting, adding or substituting an amino acid. For example, in Betaseron® the native protein has been modified by a C17S mutation. Other modifications include attachment of another protein or other chemical entities to the interferon-beta, e.g., those chemical residues which increase the water-solubility of the interferon-beta; such as straight or branched polyethylene glycol (PEG) or polypropylene glycol (PPG) moieties. In some embodiments, the interferon-beta is pegylated interferon-beta. In some embodiments, the interferon-beta is pegylated interferon-beta 1a. In some embodiments, the interferon-beta is pegylated interferon-beta 1b. In some embodiments, the interferon-beta'is formulated in a liquid formulation for injection. In some embodiments, the interferon-beta is formulated for subcutaneous injection. In some embodiments, the interferon-beta is formulated for intramuscular injection.

The term "pharmaceutically acceptable salt" means salts of the compounds of the present disclosure, which may be prepared with relatively nontoxic acids or bases, depending on the particular substituents found on the compounds described herein. When compounds of the present disclosure contain relatively acidic functionalities (e.g.,-COOH group), base addition salts can be obtained by contacting the compound (e.g., neutral form of such compound) with a sufficient amount of the desired base, either neat or in a suitable inert solvent. Examples of pharmaceutically acceptable base addition salts include lithium, sodium, potassium, calcium, ammonium, organic amino, magnesium and aluminum salts. When compounds of the present disclosure contain relatively basic functionalities (e.g., amines), acid addition salts can be obtained, e.g., by contacting the compound (e.g., neutral form of such compound) with a sufficient amount of the desired acid, either neat or in a suitable inert solvent. Examples of pharmaceutically acceptable acid addition salts include those derived from inorganic acids like hydrochloric, hydrobromic, nitric, carbonic, monohydrogencarbonic, phosphoric, diphosphoric, monohydrogenphosphoric, dihydrogenphosphoric, sulfuric, monohydrogensulfuric, hydriodic as well as the salts derived from relatively nontoxic organic acids like formic, acetic, propionic, isobutyric, malic, maleic, malonic, benzoic, succinic, suberic, fumaric, lactic, mandelic, phthalic, benzenesulfonic, p-tolylsulfonic, citric, tartaric, methanesulfonic, 2-hydroxyethylsulfonic, salicylic, stearic . Also included are salts of amino acids such as arginate, and salts of organic acids like glucuronic or galactunoric acids (see, for example, Berge et al., Journal of Pharmaceutical Science, 1977, 66: 1-19). The neutral forms of the compounds can be regenerated, for example, by contacting the salt with an acid and isolating the parent compound in the conventional manner. The parent form of the compound can differ from the various salt forms in certain physical properties, such as solubility in polar solvents, but otherwise the salts are equivalent to the parent form of the compound for the purposes of the present disclosure. Certain specific compounds of the present disclosure contain both, basic and acidic, functionalities that allow the compounds to be converted into either base or acid addition salts.

When a substituent includes a negatively charged oxygen atom "O⁻", e.g., in "COO⁻", then the formula is meant to optionally include a proton or an organic or inorganic cationic counterion (e.g., Na+). In one example, the resulting salt form of the compound is pharmaceutically acceptable. Further, when a compound of the present disclosure includes an acidic group, such as a carboxylic acid group, e.g., written as the substituent "-COOH", "CO₂H", then the formula is meant to optionally include the corresponding "de-protonated" form of that acidic group, e.g., "-COO⁻" or "-CO₂⁻", respectively.

The terms "disease" and "disorder" are used interchangeably herein.

The term "neurological disorder" refers to disorders of the nervous system that result in impairment of neuronal mediated functions and includes disorders of the central nervous system (e.g., the brain, spinal cord) as well as the peripheral nervous system. In some embodiments, the neurological disorder .is characterized by at least one of demyelination and axonal loss. The term "axonal loss" includes "axonal damage". In other embodiments, the neurological disorder is characterized by both, demyelination and axonal loss. In some embodiments, the neurological disorder is an auto-immune disease characterized by at least one of demyelination and axonal loss (e.g., MS).

The term "neuroprotection" refers to prevention or a slowing in neuronal degeneration, including, for example, demyelination and/or axonal loss, and optionally, neuronal and oligodendrocyte death.

The terms "therapeutically effective dose" and "therapeutically effective amount" refer to that amount of a compound which results in prevention or delay of onset or amelioration of symptoms of a neurological disorder in a subject or an attainment of a desired biological outcome, such as reduced neurodegeneration (e.g., demyelination, axonal loss, or neuronal death) or slowing in the accumulation of physical disability (e.g., as indicated by, e.g., a reduced rate of worsening of a clinical score (e.g., EDSS) or another suitable parameter indicating disease state. Exemplary disease state parameters include the number of clinical relapses, number of T1 lesions, reduced mean number of new and total gadolinium-enhancing (Gd+) lesions on brain MRI scans, number and volume of new or newly-enlarging T2 hyperintense lesions, number of new T1 hypointense lesions, percentage of Gd+ lesions that convert to T1 hypointense lesions, measures of atrophy and magnetization transfer ratio).

In some embodiments, the therapeutically effective dose for the compound DMF and/or MMF when used in co-therapy involving glatiramer acetate or interferon-beta (as described herein), is lower than the therapeutically effective dose for the compound DMF and/or MMF when used in monotherapy. In other embodiments, the therapeutically effective dose for glatiramer acetate, when used in co-therapy involving the compound DMF and/or MMF (as described herein), is lower than the therapeutically effective dose for glatiramer acetate, when used in monotherapy. In other embodiments, the therapeutically effective dose for interferon-beta, when used in co-therapy involving the compound DMF and/or MMF (as described herein), is lower than the therapeutically effective dose for interferon-beta, when used in monotherapy.

The term "treating" refers to administering a therapy in an amount, manner, and/or mode effective to improve a condition, symptom, or parameter associated with a disorder or to prevent progression of a disorder, to either a statistically significant degree or to a degree detectable to one skilled in the art. In some embodiments, "treating" refers to improvement of a condition as measured by a suitable clinical marker. An effective amount, manner, or mode can vary depending on the subject and may be tailored to the subject. For neurological disorders referred herein, the treatments offered by the methods disclosed herein aim at improving the conditions (or lessening the detrimental effects) of the disorders and not necessarily at completely eliminating or curing the disorders.

The term "copolymer" refers to a polypeptide consisting essentially of L-glutamic acid, L-alanine, L-tyrosine, and L-lysine as the amino acid building blocks, or pharmaceutically acceptable salts thereof. The copolymer may be a polypeptide consisting essentially of L-glutamic acid, L-alanine, L-tyrosine, and L-lysine with an average molar fraction of from about 0.05 to about 0.2 for L-glutamic acid, from about 0.1 to about 1.0 for L-alanine, from about 0.01 to about 0.2 for L-tyrosine, and from about 0.1 to about 1.0 for L-lysine. The copolymer may be a polypeptide consisting essentially of L-glutamic acid, L-alanine, L-tyrosine, and L-lysine with an average molar fraction of 0.14 for L-glutamic acid, 0.43 for L-alanine, 0.1 for L-tyrosine, and 0.34 for L-lysine. The copolymer may be a polypeptide consisting essentially of L-glutamic acid, L-alanine, L-tyrosine, and L-lysine with an average molecular weight of from 1 kD to 25 kD. The copolymer may be a polypeptide consisting essentially of L-glutamic acid, L-alanine, L-tyrosine, and L-lysine with an average molecular weight of from 3 kD to 15 kD. The copolymer may be a polypeptide consisting essentially of L-glutamic acid, L-alanine, L-tyrosine, and L-lysine with an average molecular weight of from 3 kD to 10 kD. The copolymer may be a polypeptide having the formula: (Glu, Ala, Lys, Tyr)x•xCH₃COOH, wherein the molar ratios of Glu, Ala, Lys and Tyr as described above; and x is selected so that the copolymer has an average molecular weight between 1 and 25 kD (e.g., between 4kD and 10 kD). The copolymer may be glatiramer acetate (also known as copolymer-1). Glatiramer acetate, the active ingredient. of COPAXONE®, consists of the acetate salts of synthetic polypeptides, containing four naturally occurring amino acids: L-glutamic acid, L-alanine, L-tyrosine, and L-lysine with an average molar fraction of 0.141, 0.427, 0.095, and 0.338, respectively. The average molecular weight of glatiramer acetate is 5,000 - 9,000 daltons. Glatiramer acetate is identified by specific antibodies. Chemically, glatiramer acetate is designated L-glutamic acid polymer with L-alanine, L-lysine and L-tyrosine, acetate (salt). Its structural formula is sometimes described as:

(Glu, Ala, Lys, Tyr)x•xCH₃COOH

(C₅H₉NO₄•C₃H₇NO₂•C₆H₁₄N₂O₂•C₉H₁₁NO₃)ₓ•C₂H₄O₂

CAS - 147245-92-9

COPAXONE® is a clear, colorless to slightly yellow, sterile, nonpyrogenic solution for subcutaneous injection. Each 1 mL of solution contains 20 mg of glatiramer acetate and 40 mg of mannitol. The pH range of the solution is approximately 5.5 to 7.0, The biological activity of COPAXONE® is determined by its ability to block the induction of experimental autoimmune encephalomyelitis (EAE) in mice.

COPAXONE® is indicated for reduction of the frequency of relapses in patients with Relapsing-Remitting Multiple Sclerosis (RRMS), including patients who have experienced a first clinical episode and have MRI features consistent with multiple sclerosis.

COPAXONE® is administered subcutaneously and the currently recommended dose of COPAXONE is 20 mg/day. COPAXONE is supplied as single-use prefilled syringe containing 1 mL solution with 20 mg of glatiramer acetate and 40 mg of mannitol.

AVONEX® (Interferon beta-1a) is a 166 amino acid glycoprotein with a predicted molecular weight of approximately 22,500 daltons. It is produced by recombinant DNA technology using genetically engineered Chinese Hamster Ovary cells into which the human interferon beta gene has been introduced. The amino acid sequence of AVONEX® is identical to that of natural human interferon beta.

Using the World Health Organization (WHO) natural interferon beta standard, Second International Standard for Interferon, Human Fibroblast (Gb-23-902-531), AVONEX® has a specific activity of approximately 200 million international units (IU) of antiviral activity per mg of Interferon beta-1a determined specifically by an *in vitro* cytopathic effect bioassay using lung carcinoma cells (A549) and Encephalomyocarditis virus (ECM). AVONEX® 30 mcg (micrograms) contains approximately 6 million IU of antiviral activity using this method. The activity against other standards is not known. Comparison of the activity of AVONEX® with other Interferon betas is not appropriate, because of differences in the reference standards and assays used to measure activity.

AVONEX® is supplied in vials containing 30 mcg white to off-white lyophilized powder for intramuscular injection after reconstitution with supplied diluent (Sterile Water for Injection, USP). Each vial of reconstituted AVONEX® contains 30 mcg of Interferon beta-1a; 15 mg Albumin (Human), USP; 5.8 mg Sodium Chloride, USP; 5.7 mg Dibasic Sodium Phosphate, USP; and 1.2 mg Monobasic Sodium Phosphate, USP, in 1.0 mL at a pH of approximately 7.3.

AVONEX® is also supplied in prefilled syringes formulated as a sterile liquid for intramuscular injection. Each 0.5 mL (30 mcg dose) of AVONEX® in a prefilled glass syringe contains 30 mcg of Interferon beta-1a, 0.79 mg Sodium Acetate Trihydrate, USP; 0.25 mg Glacial Acetic Acid USP; 15.8 mg Arginine Hydrochloride, USP; and 0.025 mg Polysorbate 20 in Water for Injection, USP at a pH of approximately 4.8.

AVONEX® is indicated for the treatment of patients with relapsing forms of multiple sclerosis to slow the accumulation of physical disability and decrease the frequency of clinical exacerbations. Patients with multiple sclerosis in whom efficacy has been demonstrated include patients who have experienced a first clinical episode and have MRI features consistent with multiple sclerosis. Safety and efficacy in patients with chronic progressive multiple sclerosis have not been established.

The recommended dosage of AVONEX® is 30 mcg injected intramuscularly once a week. AVONEX® is intended for use under the guidance and supervision of a physician. Patients may self-inject only if their physician determines that it is appropriate and with medical follow-up, as necessary, after proper training in intramuscular injection technique. Sites for injection include the thigh or upper arm (see Medication Guide). A 25 gauge, 1" needle for intramuscular injection may be substituted for the 23 gauge, 1 ¼" needle by the prescribing physician, if deemed appropriate.

To reconstitute lyophilized AVONEX®, use a sterile syringe and MICRO PIN® to inject 1.1 mL of the supplied diluent, Sterile Water for Injection, USP, into the AVONEX® vial. Gently swirl the vial of AVONEX® to dissolve the drug completely. Do not shake. The reconstituted solution should be clear to slightly yellow without particles. Inspect the reconstituted product visually prior to use. Discard the product if it contains particulate matter or is discolored. Each vial of reconstituted solution contains 30 mcg/1.0 mL Interferon beta-1a. Withdraw 1.0 mL of reconstituted solution from the vial into a sterile syringe. Replace the cover on the MICRO PIN®, attach the sterile needle and inject the solution intramuscularly. The AVONEX® and diluent vials are for single-use only; unused portions should be discarded

In the embodiments, the invention provides methods of treating a subject having the neurological disorder MS. In some embodiments the neurological disorder is characterized by demyelination and/or axonal loss.

Also described herein are medical uses that comprise administering to the subject:
(a) a therapeutically effective amount of at least one compound of Formula I: 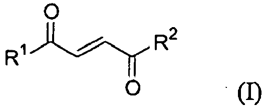 wherein R¹ and R² are independently selected from OH, O⁻, and (C₁₋₆)alkoxy, or a pharmaceutically acceptable salt thereof, and
(b) a copolymer, as described herein, or a pharmaceutically acceptable salt thereof.

In one example, in the above use, at least one of R¹ and R² is (C₁₋₆)alkoxy, In another example, in the above use, at least one of R¹ and R is methoxy. In another example, in the above use, the at least one compound of Formula I is selected from DMF, MMF, and combinations thereof. In another example, in the above use, the at least one compound of Formula I is selected from DMF and MMF. In another example, in the above use, the at least one compound of Formula I is DMF. In another example, in the above use, the at least one compound of Formula I is MMF. In another example, in the above use, the at least one compound of Formula I is a combination of DMF and MMF, In some embodiments the only compound of Formula I administered to the subject is DMF. In some embodiments the only compound of Formula I administered to the subject is MMF. In the embodiments of the invention the only compounds of Formula I administered to the subject are DMF and MMF.

In another example, in the above use, the copolymer is a polypeptide consisting essentially of L-glutamic acid, L-alanine, L-tyrosine, and L-lysine as the amino acid building blocks, or a pharmaceutically acceptable salt thereof. In another example, in the above use, the copolymer is glatiramer acetate.

Also described herein are medical uses that include administering to the subject:
(a) a therapeutically effective amount of at least one compound of Formula I: 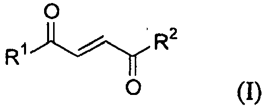 wherein R¹ and R² are independently selected from OH, O⁻, and (C₁₋₆)alkoxy, or a pharmaceutically acceptable salt thereof, and
(b) interferon-beta.

In one example, in the above use, at least one of R¹ and R² is (C₁₋₆)alkoxy, In another example, in the above use, at least one of R¹ and R² is methoxy. In another example, in the above use, the at least one compound of Formula I is selected from use DMF, MMF, and combinations thereof. In another example, in the above use, the at least one compound of Formula I is selected from DMF and MMF. In another example, in the above use, the at least one compound of Formula I is DMF. In another example, in the above use, the at least one compound of Formula I is MMF. In another example, in the above use, the at least one compound of Formula I is a combination of DMF and MMF. In some embodiments the only compound of Formula I administered to the subject is DMF. In some embodiments the only compound of Formula I administered to the subject is MMF. In the embodiments of the invention the only compounds of Formula I administered to the subject are DMF and MMF.

In some embodiments in which DMF is administered to the subject, the DMF is formulated in capsules containing enterically coated microtablets. In some embodiments, the coating of the microtablets is composed of different layers. In some embodiments, the first layer is a methacrylic acid - methyl methacrylate copolymer/isopropyl alcohol solution which isolates the tablet cores from potential hydrolysis from the next applied water suspensions. Enteric coating of the tablet is then conferred by an aqueous methacrylic acid-ethyl acrylate copolymer suspension. An exemplary formulation for DMF is described in Example 7. Additional methods of synthesizing and formulating the copolymer (e.g., DMF and MMF) are provided, for example, in the Examples at columns 5-7 of U.S. Patent No. 7,320,999.

Neurological disorders (e.g., those characterized by demyelination and/or axonal loss) include by way of example and without limitation: Multiple sclerosis, Huntington's disease, Alzheimer's disease, Parkinson's disease, optic neuritis, Devic disease, transverse myelitis, acute disseminated encephalomyelitis, adrenoleukodystrophy and adrenomyeloneuropathy, acute inflammatory demyelinating polyneuropathy (AIDP), chronic inflammatory demyelinating polyneuropathy (CIDP), acute transverse myelitis, progressive multifocal leucoencephalopathy (PML), acute disseminated encephalomyelitis (ADEM) or other hereditary disorders (e.g., leukodystrophies, Leber's optic atrophy, and Charcot-Marie-Tooth disease).

In some embodiments of the disclosure, a patient having a neurological disorder characterized by demyelination and/or axonal loss is treated. For example, the degree of demyelination and/or axonal loss may be such as present in a patient with a score of 3, 3.5, 4, 4.5, 5, 5.5, 6, 6.5, 7 or higher on the Expanded Disability Status Scale (EDSS; see Table 1 below). Other suitable measurement scales can also be used (see, e.g., pp. 288-291 in McAlpine's Multiple Sclerosis, by Alastair Compston et al., 4th edition, Churchill Livingstone Elsevier, 2006).

**Table 1. Expanded Disability Status Scale (EDSS)**

| | |
|---|---|
| **0** | Normal neurological examination (all grade 0 in functional systems [FS]; cerebral grade 1 acceptable) |
| **1** | No disability, minimal signs in 1 FS (i.e. grade 1 excluding cerebral grade 1) |
| **1.5** | No disability, minimal signs in >1 FS (>1 grade 1 excluding cerebral grade 1) |
| **2** | Minimal disability in 1 FS (1 FS grade 2, others 0 or 1) |
| **2.5** | Minimal disability in 2 FS (2 FS grade 2, others 0 or 1) |
| **3** | Moderate disability in 1 FS (1 FS grade 3, others 0 or 1), or mild disability in 3-4 FS (3-4 FS grade 2, others 0 or 1) though fully ambulatory |
| **3.5** | Fully ambulatory but with moderate disability in 1 FS (1 FS grade 3) and 1-2 FS grade 2; or 2 FS grade 3; or 5 FS grade 2 (others 0 or 1) |
| **4** | Fully ambulatory without aid, self-sufficient, up and about some 12 hours a day despite relatively severe disability consisting of 1 FS grade 4 (others 0 or 1), or combinations of lesser grades exceeding limits of previous steps. Able to walk without aid or rest some 500 m |
| **4.5** | Fully ambulatory without aid, up and about much of the day, able to work a full day, may otherwise have some limitation of full activity or require minimal assistance; characterized by relatively severe disability, usually consisting of 1 FS grade 4 (others 0 or 1) or combinations of lesser grades exceeding limits of previous steps. Able to walk without aid or rest for some 300 m |
| **5** | Ambulatory without aid or rest for about 200 m; disability severe enough to impair full daily activities (e.g. to work full day without special provisions). (Usual FS equivalents are 1 grade 5 alone, others 0 or 1; or combination of lesser grades usually exceeding specifications for step 4.0) |
| **5.5** | Ambulatory without aid or rest for about 100 m, disability severe enough to preclude full daily activities. (Usual FS equivalents are 1 grade 5 alone, others 0 or 1; or combination of lesser grades usually exceeding those for step 4.0) |
| **6** | Intermittent or unilateral constant assistance (cane, crutch or brace) required to walk about 100 m with or without resting. (Usual FS equivalents are combinations with >2 FS grade 3+) |
| **6.5** | Constant bilateral assistance (canes, crutches or braces) required to walk about 20 m without resting. (Usual FS equivalents are combinations with >2 FS grade 3+) |
| **7** | Unable to walk beyond about 5 m even with aid, essentially restricted to wheelchair; wheels self in standard wheelchair and transfers alone; up and about in wheelchair some 12 hours a day. (Usual FS equivalents are combinations with >1 FS grade 4+; very rarely, pyramidal grade 5 alone) |
| **7.5** | Unable to take more than a few steps; restricted to wheelchair, may need aid in transfer; wheels self but cannot carry on in standard wheelchair a full day; may require motorized wheelchair. (Usual FS equivalents are combinations with >1 FS grade 4+) |
| **8** | Essentially restricted to bed or chair or perambulated in wheelchair, but may be out of bed itself much of the day; retains many self-care functions; generally has effective use of arms. (Usual FS equivalents are combinations, generally 4+ in several systems) |
| **8.5** | Essentially restricted to bed much of the day; has some effective use of arm(s); retains some self-care functions. (Usual FS equivalents are combinations, generally 4+ in several systems) |
| **9** | Helpless bedridden patient; can communicate and eat. (Usual FS equivalents are combinations, mostly grade 4+) |
| **9.5** | Totally helpless bedridden patient; unable to communicate effectively or eat/swallow. (Usual FS equivalents are combinations, almost all grade 4+) |
| **10** | Death due to multiple sclerosis |

As another example, the degree of demyelination and/or axonal loss may be such as that in a patient who has more than 10, 12, 15, 20 or more hypointense T1 lesions. The number of such lesions can be determined, for example, by routine MRI methods.

As described herein, the subject may have a progressive form of a demyelinating disorder, e.g., MS (e.g., primary progressive MS or secondary progressive MS) or Devic's disease. In some cases, as for example, in secondary progressive MS, the subject may have a disorder that may be characterized by initial inflammation followed by progressive demyelination and/or axonal loss. The diagnosis of MS may be performed as per McDonald's criteria as described in, e.g., McDonald et al., Ann. Neurol., 2001, 50:120-127; or the 2005 revised criteria as described in, e.g., Polman et al., Annals of Neurology, 2005, 58(6):840-846.

In some embodiments, the subject being treated has secondary progressive MS and an EDSS score of more than 5, 5.5, 6, 6.5, 7, or higher.

The disease progression in the subject can be such that the subject exhibits at least a 1-, 1.5-, 2-, 2.5-, 3-, 3.5-point or greater increase in the EDSS score in the previous year and/or at least a 25%, 30%, 40%, 50%, 75%, or 100% increase in T1 lesion load over the previous year.

Additional parameters describing the subjects with an advanced stage demyelinating disorder can be (a) T2 lesion volume of more than 15 cm³ and/or (b) corpus callosum area of less than 400 mm².

In certain embodiments, the combination therapies for use in treating a subject provide treated subjects neuroprotective effects, e.g., protection of the neuronal cells or nerve processes (axons) from death or being damaged. These neuroprotective effects do not necessarily eliminate all of the damages or degeneration, but rather, delay or even halt the progress of the degeneration or a prevention of the initiation of the degeneration process or an improvement to the pathology of the disorder. In some embodiments the combination therapies for use in treating a subject offer neuroprotection to at least one part of the nervous system, such as for example the central nervous system, e.g., hippocampus, cerebellum, spinal cord, cortex (e.g., motor or somatosensory cortex), striatum, basal forebrain (cholenergic neurons), ventral mesencephalon (cells of the substantia nigra), and the locus ceruleus (neuroadrenaline cells of the central nervous system).

In some embodiments of the combination therapies for use in treating a subject the subject being treated is a subject in need of neuroprotection, including subjects who have extensive demyelination and/or axonal loss such as subjects that have secondary progressive MS or another demyelinating disorder as specified above. In some embodiments of the combination therapies for use in treating a subject the subjects are mammalian, e.g., rodents or another laboratory animal, e.g., a non-human primate. In some embodiments, the subject is human. In some embodiments, the human subject is older than 55, 57, 60, 65, or 70 years of age. Also described herein are combination therapies for use in treating a subject comprising (A) administering to a test animal, i.e., rodent (e.g., mouse) (a) a compound of Formula I, as described herein, wherein R¹ and R² are independently selected from OH, O⁻, and (C₁₋₆)alkoxy, or a pharmaceutically acceptable salt thereof, and (b) glatiramer acetate or interferon-beta; and (B) measuring a mean clinical score according to an experimental autoimmune encephalomyelitis model, e.g., chronic oligodendrocyte glycoprotein induced experimental autoimmune encephalomyelitis model (MOG-EAE); e.g., as described herein.

In some embodiments, DMF and/or MMF and glatiramer acetate or interferon-beta are administered in an amount and for a period of time sufficient to reduce demyelination and/or axonal death in the subject.

In some embodiments, DMF and/or MMF and glatiramer acetate or interferon-beta are administered in an amount and for a period of time sufficient to slow the accumulation of disability, e.g., progression in disability, in the subject. In some embodiments, DMF and/or MMF and glatiramer acetate or interferon-beta are administered in an amount and for a period of time sufficient to decrease the rate of relapse. Accumulation of disability/progression in disability is reflected by, for example, an increase in the EDSS score and may be measured as the length of time to an increase of at least 1 point in the EDSS score. For example, the compound of Formula I and glatiramer acetate or interferon-beta may be administered in an amount and for a period of time sufficient to sustain an increase in the EDSS score within 1 point or less for 3, 4, 5, 6, 7, 8, 9, 10,11, 12, 24, 36 months or longer.

In some embodiments the combination therapy for use in treating a subject includes treating the subject with a therapeutically effective amount of at least one compound chosen from DMF and MMF. For DMF or MMF, the therapeutically effective amount can range from 1 mg/kg to 50 mg/kg (e.g., from 2.5 mg/kg to 20 mg/kg or from 2.5 mg/kg to 15 mg/kg). Effective doses will also vary, as recognized by those skilled in the art, dependent on route of administration, excipient usage, and the possibility of co-usage with other therapeutic treatments including use of other therapeutic agents. For example, an effective dose of DMF or MMF to be administered to a subject, for example orally, can be from 0.1 g to 1 g per day, for example, from 100 mg to 800 mg per day (e.g., from 120 mg to 740 mg per day, from 240 mg to 720 mg per day; or from 480 mg to 720 mg per day; or 720 mg per day). An effective dose of DMF or MMF can also be, for example, 100 mg per day, 200, mg per day, 300 mg per day, 400 mg per day, 500 mg per day, 600 mg per day, 700 mg per day, 800 mg per day, 900 mg per day, or 1 g per day. The dosages may be administered one or more times per day. For example, 720 mg per day may be administered all at once or in separate administrations of 2, 3, 4, 5 or 6 (e.g., equal) doses.

In some embodiments a therapeutically effective amount of at least one of DMF or MMF and a therapeutically effective amount of glatiramer acetate is used. In some embodiments, the therapeutically effective amount for glatiramer acetate in the use of the invention can range from 1 mg per day to 100 mg per day. In other embodiments, the therapeutically effective amount of glatiramer acetate can range from
5 mg per day to 50 mg per day. In other embodiments, the therapeutically effective amount of glatiramer acetate can range from 5 mg per day to 30 mg per day, or from 10 mg per day to 30 mg per day. In yet other embodiments, the therapeutically effective amount of glatiramer acetate can range from 15 mg per day to 25 mg per day. In other embodiments, the therapeutically effective amount of glatiramer acetate is 20 mg per day. In other embodiments, the therapeutically effective amount of glatiramer acetate is less than the therapeutically effective amount when used in mono-therapy (e.g., 20 mg per day). In other embodiments, the therapeutically effective amount of glatiramer acetate is less than 20 mg per day. For example, the therapeutically effective amount of glatiramer acetate can range from about 5 mg per day to 19 mg per day, or from 10 mg per day to 19 mg per day or from 15 mg per day to 19 mg per day. Any of the above amounts of glatiramer acetate can be administered all at once or in separate administrations of 2, 3, 4, 5 or 6 (e.g., equal) doses.

In some embodiments the combination therapies for use in treating a subject include a therapeutically effective amount of at least one compound of DMF or MMF and a therapeutically effective amount of interferon-beta. In some embodiments, the therapeutically effective amount for interferon-beta in the methods of the invention can range from 10 mcg per week to 500 mcg per week. In other embodiments, the therapeutically effective amount of interferon-beta can range from 20 mcg per week to 300 mcg per week. In other embodiments, the therapeutically effective amount of interferon-beta can range from 20 mcg per week to 200 mcg per week. In other embodiments, the therapeutically effective amount of interferon-beta can range from 20 mcg per week to 100 mcg per week. In other embodiments, the therapeutically effective amount of interferon-beta can range from 20 mcg per week to 80 mcg per week, or from 20 mcg per week to 60 mcg per week. In other embodiments, the therapeutically effective amount of interferon-beta can range from 20 mcg per week to 40 mcg per week. In other embodiments, the therapeutically effective amount of interferon-beta is 30 mcg per week. In other embodiments, the therapeutically effective amount of interferon-beta is less than the therapeutically effective amount when used in mono-therapy (e.g., 30 mcg per week). In other embodiments, the therapeutically effective amount of interferon-beta is less than 30 mcg per week. For example, the therapeutically effective amount of interferon-beta can range from 5 mcg per week to 29 mcg per week, or from 10 mcg per week to 29 mcg per week, or from 15 mcg per week to 29 mcg per week, or from 20 mcg per week to 29 mcg per week. Any of the above amounts of interferon-beta can be administered all at once or in separate administrations of 2, 3, 4, 5 or 6 (e.g., equal) doses. In some embodiments, the interferon-beta is administered at a dose of 5 mcg to 80 mcg at one time.

In some embodiments in which the interferon-beta is interferon-beta 1a, interferon-beta 1a is administered (e.g., subcutaneously or intramuscularly) at a dose of 5 mcg to 80 mcg at one time. In some examples, interferon-beta 1a is administered at a dose of 20 mcg to 46 mcg at one time. In some embodiments, interferon-beta 1a (e.g., Avonex®) is administered (e.g., intramuscularly) once a week. In some embodiments, interferon-beta 1a (e.g., Avonex®) is administered (e.g., intramuscularly) at a dose of 30 mcg once 1a week. In some embodiments, the interferon-beta 1a (e.g., Avonex®) is administered (e.g., intramuscularly) at a dose of less than 30 mcg once a week.

In some embodiments, interferon-beta 1a is administered (e.g., subcutaneously) twice a week equivalent to a total dose of from 10 mcg to 160 mcg per week, or 40 mcg to 92 mcg per week. In some embodiments, interferon-beta 1a (e.g., Rebif®) is administered (e.g., subcutaneously) three times a week equivalent to a total dose of from 15 mcg to 240 mcg per week, or 60 mcg to 138 mcg per week.

In some embodiments in which the interferon-beta is interferon-beta 1b, interferon-beta 1b is administered (e.g., subcutaneously or intramuscularly) at a dose of 50 mcg to 400 mcg at one time. In some examples, interferon-beta 1b, is administered at a dose of 50 mcg to 300 mcg at one time. In some examples, interferon-beta 1b is administered at a dose of 200 mcg to 300 mcg at one time. In some embodiments, interferon-beta 1b is administered once a week. In some embodiments, interferon-beta 1b is administered twice a week equivalent to a total dose of from 100 mcg to 800 mcg per week. In some embodiments, interferon-beta 1b is administered three times a week equivalent to a total dose of from 300 mcg to 1200 mcg per week. In some embodiments, interferon-beta 1b (e.g., Betaseron®) is administered (e.g., subcutaneously) every other day. In some embodiments, interferon-beta 1b (e.g., Betaseron®) is administered (e.g., subcutaneously) at a dose of 200 to 300 mcg every other day. In some embodiments, interferon-beta 1b (e.g., Betaseron®) is administered (e.g., subcutaneously) at a dose of 250 mcg every other day. In some embodiments, the interferon-beta 1b (e.g., Betaseron®) is administered (e.g., subcutaneously) at a dose of less than 250 mcg every other day.

The therapeutic compound of Formula 1 (DMF and/or MMF and glatiramer acetate or interferon-beta can be administered by any method that permits the delivery of the agents for treatment of neurological disorders. For instance, the compound of Formula I and the glatiramer acetate can be administered via pills, tablets, microtablets, pellets, micropellets, capsules (e.g., containing microtablets), suppositories, liquid formulations for oral administration, and in the form of dietary supplements. In some embodiments the compound of Formula I and the glatiramer acetate are formulated into a single dosage form for administration together, while in other embodiments the compound of Formula I and the glatiramer acetate are formulated into separate dosage forms for administration separately or together. In one example when the compound of Formula 1 and the glatiramer acetate are administered separately, they are administered at the same time. In another example when the compound of Formula 1 and the glatiramer acetate are administered separately, they are administered at different times.

The pharmaceutically acceptable compositions can include well-known pharmaceutically acceptable excipients, e.g., if the composition is an aqueous solution containing the active agent, it can be an isotonic saline, 5% glucose, or others. Solubilizing agents such as cyclodextrins, or other solubilizing agents well known to those familiar with the art, can be utilized as pharmaceutical excipients for delivery of the therapeutic compound. See, e.g., US Patent Nos. 6,509,376 and 6,436,992, incorporated for some formulations containing DMF. As to the route of administration, the compositions can be administered orally, intranasally, transdermally, subcutaneously, intradermally, vaginally, intraaurally, intraocularly, intramuscularly, buccally, rectally, transmucosally, or via inhalation, or intravenous administration. In some embodiments DMF is administered orally.

The interferon-beta can be formulated for administration by injection. For example, in some embodiments the interferon-beta is administered by intramuscular injection while in other embodiments it is administered by subcutaneous injection or intravenously.

In some embodiments, the method comprises administering orally a capsule containing a pharmaceutical preparation consisting essentially of 60-240 mg (e.g., 120 mg) of dimethyl fumarate in the form of enteric-coated microtablets. In some embodiments, the mean diameter of such microtablets is 1-5 mm, e.g., 1-3 mm or 2 mm.

The compound of Formula I can be administered in the form of a sustained or controlled release pharmaceutical formulation. Such formulation can be prepared by various technologies by a skilled person in the art. For example, the formulation can contain the therapeutic compound, a rate-controlling polymer (i.e., a material controlling the rate at which the therapeutic compound is released from the dosage form) and optionally other excipients. Some examples of rate-controlling polymers are hydroxy alkyl cellulose, hydroxypropyl alkyl cellulose (e.g., hydroxypropyl methyl cellulose, hydroxypropyl ethyl cellulose, hydroxypropyl isopropyl cellulose, hydroxypropyl butyl cellulose and hydroxypropyl hexyl cellulose), poly(ethylene)oxide, alkyl cellulose (e.g., ethyl cellulose and methyl cellulose), carboxymethyl cellulose, hydrophilic cellulose derivatives, and polyethylene glycol, compositions, e.g., those described in WO 2006/037342.

The following examples are illustrative and do not limit the scope of the disclosure or the claims.

### EXAMPLES

### Example 1:

### Efficacy of DMF in MOG-EAE

DMF was tested and shown to be effective in chronic oligodendrocyte glycoprotein induced experimental autoimmune encephalomyelitis (MOG-EAE) and to suppress macrophage infiltration without suppressing T-cell infiltration. See also, Schilling et al., "Fumaric Acid Esters Are Effective in Chronic Experimental Autoimmune Encephalomyelitis and Supress Macrophage Infiltration," Clinical and Experimental Immunology, Vol. 145, No. 1, pp. 101-107 (2006).

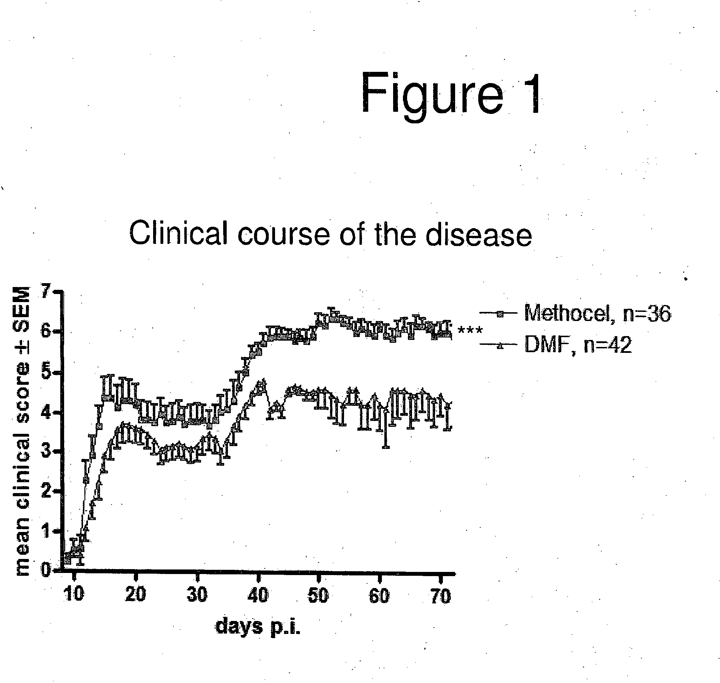

Figure 1 compares the mean clinical score in 36 mice treated with control (Methocel carrier) to 42 mice treated with DMF 15 mg/kg twice daily via oral gavage. As shown, the mean clinical score was reduced by DMF treatment.

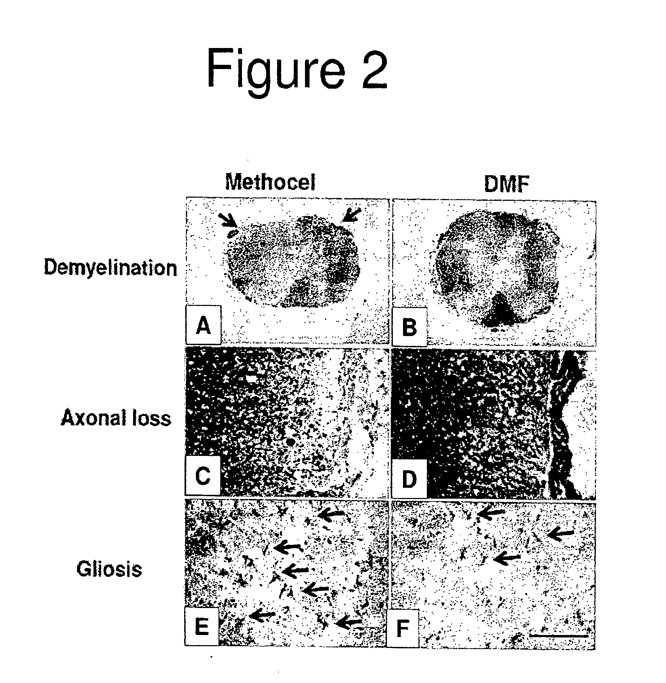

Figure 2A shows demyelination in a mouse MOG-EAE model in a control mouse. Figure 2B shows that demyelination was reduced by administration of DMF.
Figure 2C shows the level of relative axonal density in a mouse MOG -EAE model in a control mouse. Figure 2D shows that axonal loss was reduced in the animals treated with DMF.
Figures 2E and 2F show gliosis.

### Example 2:

### Titration of Glatiramer Acetate in MOG-EAE

Glatiramer acetate was tested and shown to synergize with atorvastatin in an MOG -EAE model system to prevent clinical and histological signs of EAE. See also, Stüve et al., "Immunomodulatory synergy by combination of atorvastatin and glatiramer acetate in treatment of CNS autoimmunity," J Clin. Invest., Vol. 116, No. 4, pp. 1037-44 (2006).

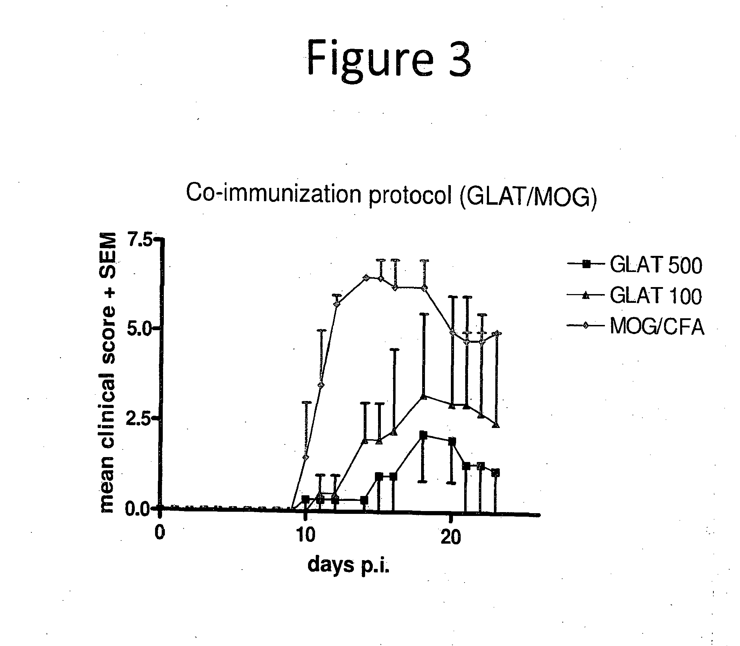

In this experiment mice were co-injected with glatiramer acetate and the MOG antigen. Specifically, a control group received MOG alone, while two experimental groups received doses of 100 or 500 mcg glatiramer acetate. As shown in Figure 3, glatiramer acetate reduced the mean clinical score of the 100 or 500 mcg groups in a dose-dependent manner. Based on the results of this experiment a dose of 50 mcg was chosen for use in combination experiments.

### Example 3:

### DMF and Glatiramer Acetate Combination Therapy in Mice

A combination of DMF and glatiramer acetate was tested for its effect on EAE symptoms. The treatment groups were as follows:
1) Mice immunized with 200µg MOG/CFA (complete Freund's adjuvant) + 200ng PTX (pertussis toxin) + vehicle (methocel).
2) Mice immunized with 200µg MOG/CFA and 50µg GA + vehicle.
3) Mice immunized with 200µg MOG/CFA; treatment with DMF (15mg/kgBW).
4) Mice immunized with 200µg MOG/ 50µg GA/CFA; treatment with DMF (15mg/kgBW).

Long term experiment: additional boost on day 28 p.i.

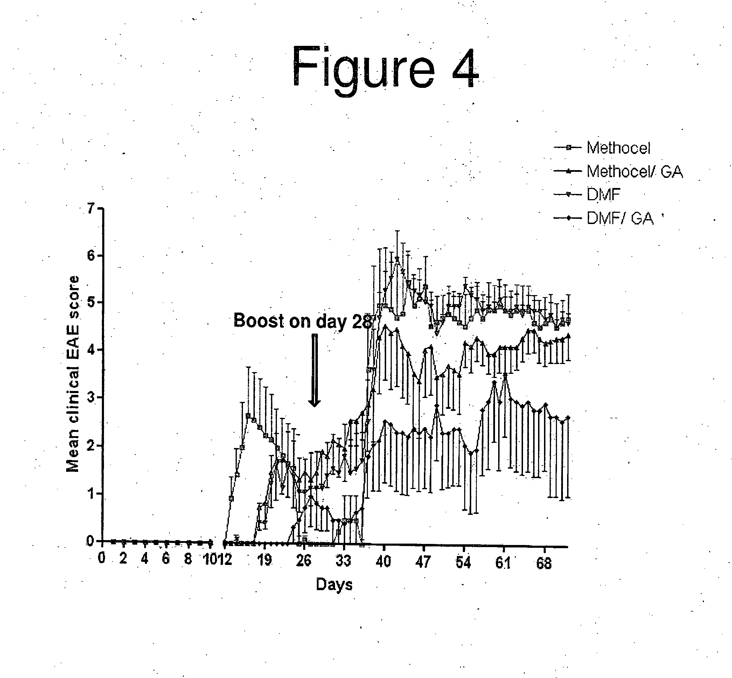

Figure 4 shows the results of a DMF and GA co-therapy during chronic MOG-EAE. Incidence: methocel (6/6), methocel/GA (6/6), DMF (6/6), DMF/GA (3/6). As shown, in the figure, at this dose both GA and DMF delayed the onset of EAE symptoms. However, DMF had little if any effect on the mean clinical EAE score during the chronic phase of the disease, whereas GA had an intermediate effect at that stage. In contrast, the combination of DMF with GA reduced the mean clinical EAE score below that observed when GA was administered as monotherapy.

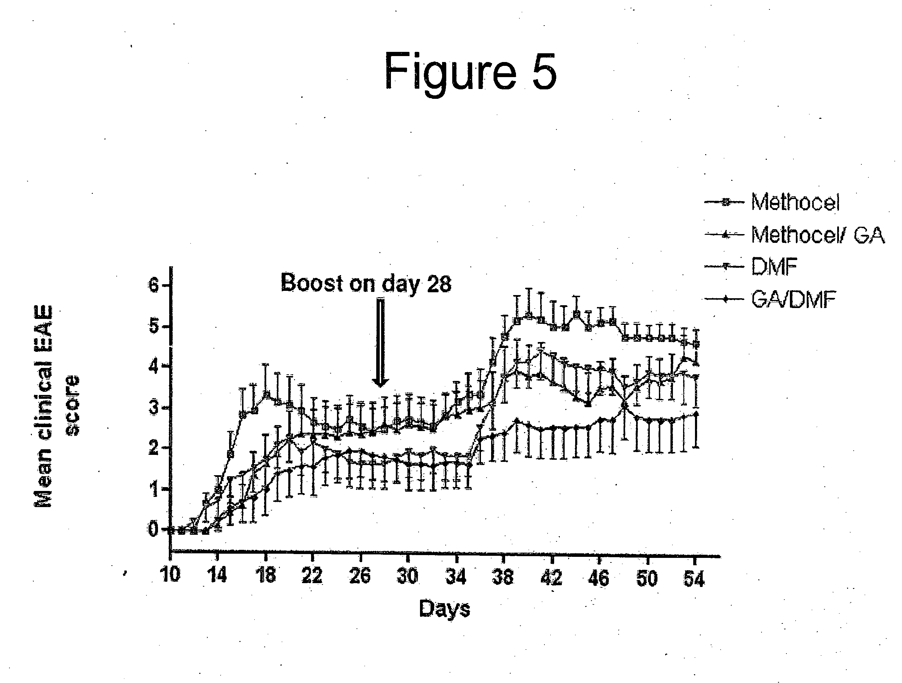

Figure 5 presents pooled data from two experiments (n= 12 per group). Incidence: methocel (12/12), methocel/GA (11/12), DMF (9/12), DMF/GA (8/12).

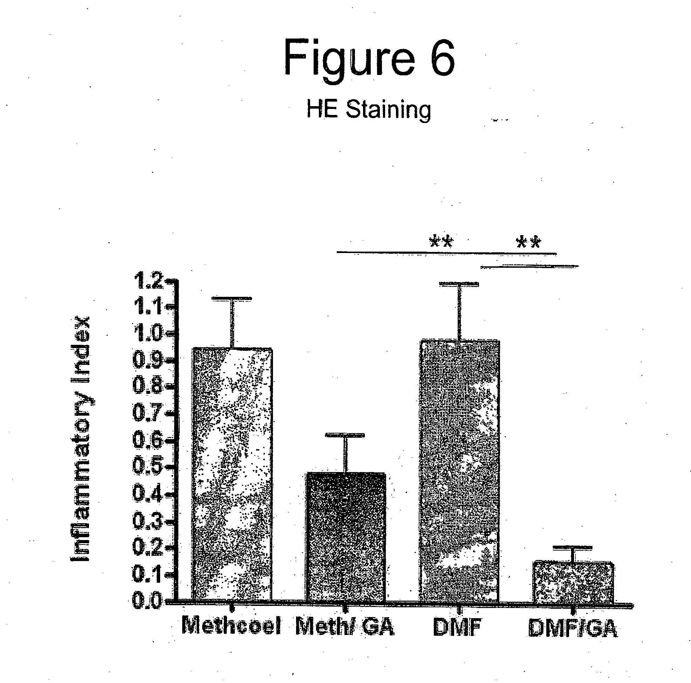

Figure 6 shows histological analysis (HE staining) of spinal cords of the different treatment groups during the chronic disease phase. As shown in the figure, GA monotherapy reduces infiltrates (as reflected by the lower inflammatory index) while DMF monotherapy does not. The combination of DMF with GA shows an even greater reduction in infiltrates than that observed with GA alone.

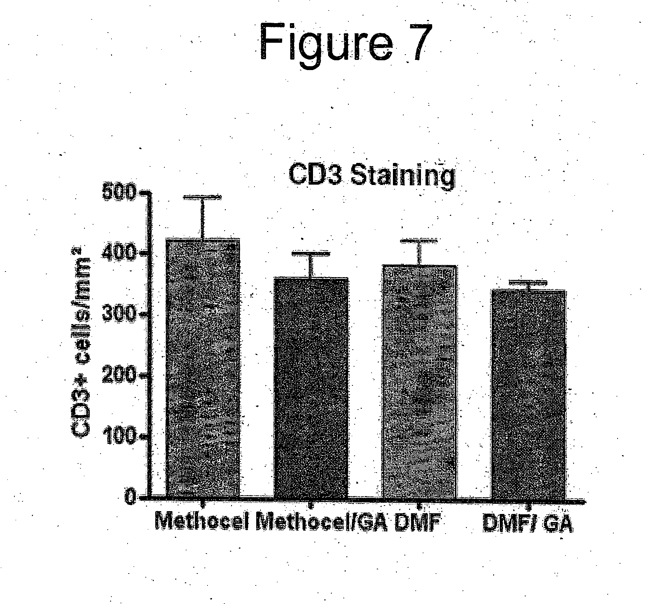

Figure 7 shows that neither GA nor DMF reduces infiltration by T cells.

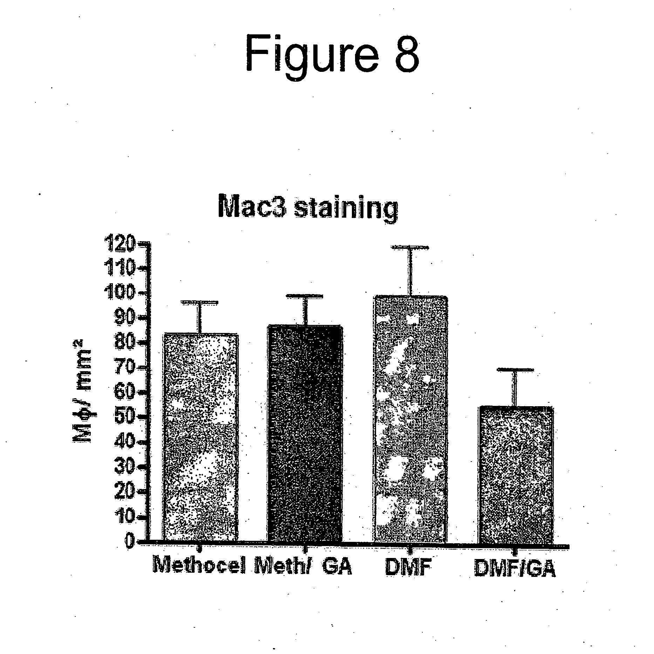

Figure 8 shows that the combination of GA with DMF results in a reduction in the number of infiltrating macrophages, whereas at the doses tested neither GA nor DMF alone does so.

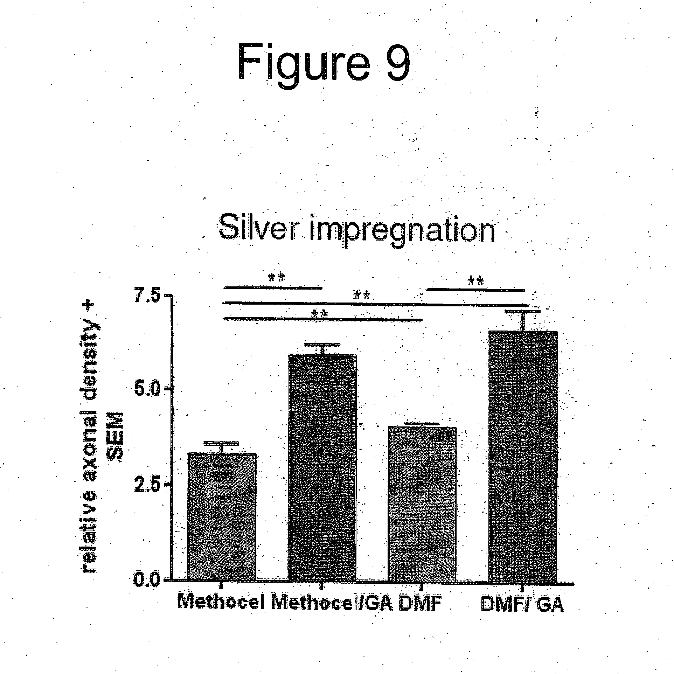

Figure 9 shows that both GA and DMF monotherapy reduce axonal loss and destruction, whereas the combination of GA with DMF does so to an even greater degree.

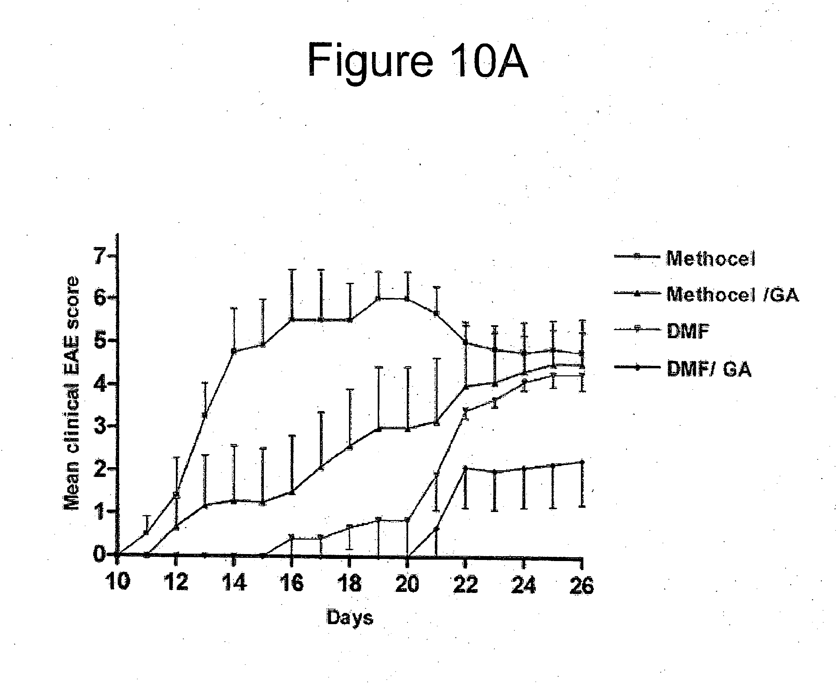

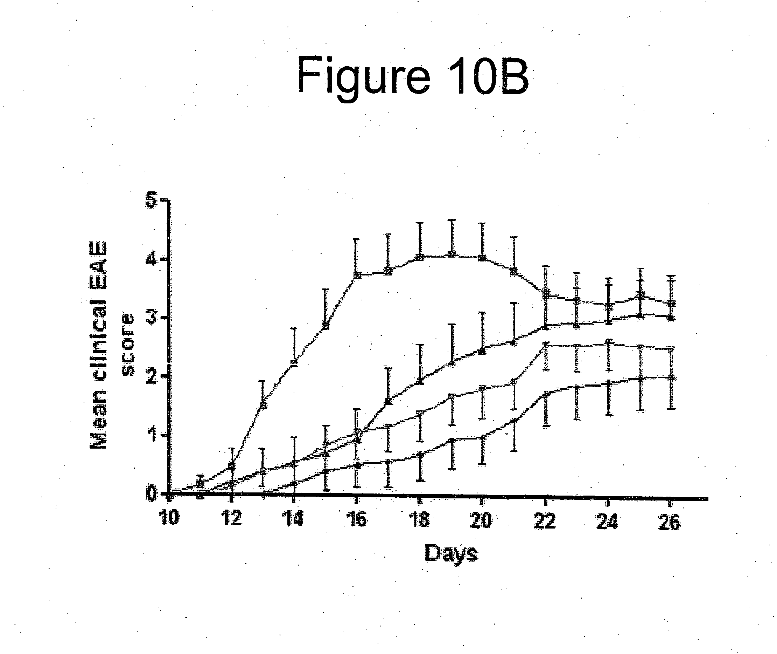

Figure 10 shows the results of DMF and GA co-therapy during the short term course of MOG-EAE. The presented data are for a single representative experiment (Figure 10A) and pooled data from three experiments (Figure 10B). As can be seen, the activity of DMF is greater than that of GA, while the combination of DMF with GA leads to an even greater reduction in the onset and level of the mean clinical EAE score. Incidence: methocel (15/18), methocel/GA (13/18), DMF (11/18), DMF/GA (9/9).

### Example 4:

### DMF and Interferon-Beta Combination Therapy in Mice

Interferon-beta has been used to treat relapsing remitting forms of MS and is known to reduce risk of disability progression. Interferon-beta is thought to act via at least one of the following mechanisms: reduction of the number and size of active MRI lesions; inhibition of T cell migration (via inhibition of MMPs); immunomodulation (shift from pro-inflammatory Th1 to anti-inflammatory Th2 response); inhibition of IL-17 production in CD4+ cells; influence on NF-κB signaling pathways; and IFNAR signaling on monocytes.

### Treatment Groups:

1) Methocel p.o. and NaCl 0.9% s.c.
2) DMF 15mg/kg p.o. and NaCl 0.9% s.c.
3) Methocel p.o. and IFN-beta s.c. (10.000 U e.o.d)
4) DMF 15mg/kg p.o. and IFN-beta s.c (10.000 U e.o.d)

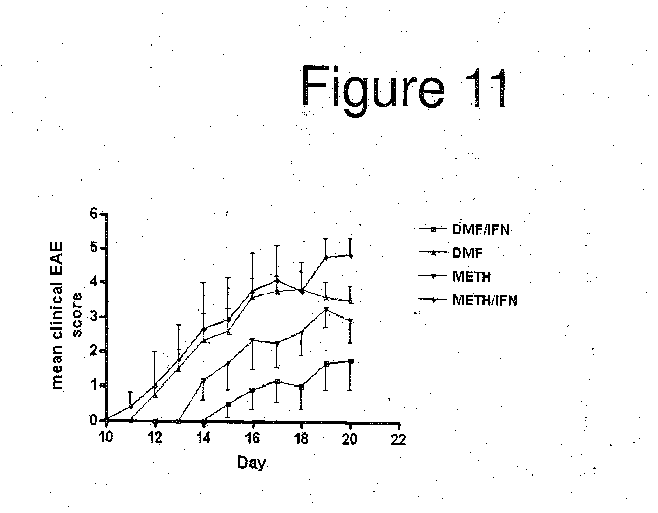

Figure 11 shows the results of administration of DMF and IFN-beta to MOG-EAE mice. Incidence: methocel (5/6), methocel/IFN-beta (5/6), DMF (6/6), DMF/IFN-beta (3/6). As shown in the figure, over a short-term course of EAE the tested doses of DMF and IFN-beta both exacerbated the EAE compared to the methocel controls. In contrast, the combination of DMF with IFN-beta delayed on set of EAE symptoms and lessened the severity of those symptoms as measured by the mean clinical EAE score.

The data presented herein, see e.g. examples 3 and 4, show that combinations of GA or IFN-beta with DMF enhance the clinical effect of GA or DMF alone. In the acute and chronic phases of MOG-EAE combination of GA with DMF. leads to a better preservation of axons than treatment with GA or DMF alone. Additional effects on immune cell infiltration are less prominent (mainly mediated by GA). In the acute phase of MOG-EAE combination of DMF with IFN-beta enhances the clinical efficacy of DMF.

Example 5:

### Treatment of Patients with Relapsing Remitting Multiple Sclerosis With DMF and Beta Interferons or Glatiramer Acetate (GA)

At the time of this application, a Phase 2, open-label, multicenter study has been initiated to be conducted in about 100 patients with relapsing-remitting MS (RRMS).

Subjects receiving an IFN-beta (Avonex®, Rebif®, or Betaseron®) or GA as monotherapy are being enrolled. Safety, efficacy, and pharmacodynamics (PD) are being assessed during a monotherapy period (IFN-beta or GA) and a subsequent 6 month combination treatment period with DMF 240 mg TID (DMF 240 mg TID demonstrated efficacy and a favorable safety profile in a Phase 2b study, and is currently being evaluated in two large Phase 3 studies). This dose allows for a thorough assessment for potential combination safety signals. To improve tolerability, DMF is being administered at a starting dose of 120 mg TID for 1 week. After 1 week, subjects receive DMF 240 mg TID for the remainder of the combination period.

Inclusion Criteria: Aged 18 to 55 years old, inclusive, at the time of informed consent; must have a confirmed diagnosis of RRMS according to McDonald criteria #1-4 (see, e.g., Polman et al., Ann Neurol 58(6):840-846 (2005), incorporated herein by reference), and have a prior brain MRI demonstrating lesion(s) consistent with MS from any point in time; must have an EDSS between 0.0 and 5.0, inclusive; must be taking the same dose of a prescribed IFN-beta (either Avonex®, Betaseron®, Rebif®) or GA for at least 12 months consecutively at the time of enrollment and remain on this treatment for the duration of the study. Subjects receiving Rebif must be prescribed 44 µg by subcutaneous injection three times per week.

Exclusion Criteria: primary progressive, secondary progressive, or progressive relapsing MS (e.g., as defined by Polman et al., Ann Neurol 58(6):840-846 (2005)); other chronic disease, of the immune system, malignancies, acute urologic, pulmonary, gastrointestinal disease; pregnant or nursing women; participation within 6 months prior to study enrollment in any other drug, biologic, or device study.

The primary objective of the study is to evaluate the safety and tolerability of DMF administered in combination with IFN-beta or GA in subjects with RRMS.

Additional objectives of the study are to explore the efficacy of DMF in combination with IFN-beta or GA, and to explore the effect of combination therapy on potential biomarkers of DMF and neopterin for IFN-beta and IFN-beta with DMF.

The primary endpoint of the study is to evaluate safety and tolerability by the incidence and type of adverse events (AEs), serious adverse events, AEs leading to discontinuation of study treatment, the incidence and type of laboratory abnormalities, and MS disease activity by magnetic resonance imaging (MRI) in all subjects.

Additional endpoints are:
Efficacy: mean number of new and total gadolinium-enhancing (Gd+) lesions on brain MRI scans; number and volume of new or newly-enlarging T2 hyperintense lesions and new T1 hypointense lesions; percentage of Gd+ lesions that convert to T1 hypointense lesions; measures of atrophy and magnetization transfer ratio; number of clinical relapses; disability status will be assessed by the Expanded Disability Status Scale (EDSS).
Health Outcomes: change in physical function as measured by Multiple Sclerosis Impact Scale (MSIS-29); change in mental function as measured by MSIS-29; change in quality of life (QoL) as measured by the Short Form Health Survey (SF-36) and the European Quality of Life (EQ)-5 Dimensions (EQ-5D), which includes the EQ-Visual Analog Scale (EQ-VAS); change in Health Resource Utilization (HRU) as measured by: 1) number of hospitalizations and emergency room (ER) visits; 2) number of unplanned visits to the neurologist due to relapse; 3) patient-reported Health Care Treatment Form which includes the number of other unplanned visits to the neurologist for non-relapse related reasons, number of visits to other physicians (e.g., urologist) or non-physician healthcare providers (e.g., physical therapist).
Pharmacodynamic: exploratory evaluations of Nrf2 pathway markers and other anti-inflammatory markers that may respond to treatment with DMF at protein and/or ribonucleic acid (RNA) level; changes in neopterin concentration for subjects on IFN-beta and IFN-beta with DMF therapy.

Subjects are grouped according to their current therapy. There are 2 treatment groups comprising approximately 50 subjects each as follows: Group 1 (IFN-beta) and Group 2 (GA). After screening and upon enrollment, these subjects continue on their prescribed treatments for 2 months. After 2 months of monotherapy, all groups receive 240 mg DMF three times daily (TID) in combination with their existing MS monotherapy for an additional 6 months. (During the first week of DMF dosing, 120 mg TID is administered. After 1 week, daily dosing escalates to 240 mg DMF TID).

The total duration of the treatment period is approximately 8 months. The visit schedule consists of a Screening Visit; monotherapy visits monthly (week -8 [enrollment] and week -4); combination therapy visits monthly (weeks 0 [baseline], 4, 8, 12, 16, 20, and 24); and a follow-up visit (approximately 14 days after the last dose of DMF). Should a subject experience a relapse, a relapse assessment visit occurs (Unscheduled Relapse Assessment Visit).

Safety assessments include physical examination, vital signs, 12-lead ECG, blood chemistry, hematology, urinalysis, AE monitoring, and MS disease activity by MRI in all subjects.

Efficacy is assessed based on changes in brain MRI, with and without Gd, (T2 hyperintense lesions, T1 hypointense lesions, Gd+ lesions, brain atrophy) and clinical relapse rate is determined. Disability status is measured by EDSS.

### Example 6:

### Pharmacokinetics of DMF Alone and With IFN-Beta-1A or Glatiramer Acetate in Healthy Volunteers

To assess the potential drug interaction for co-administration of DMF + intramuscular (IM) IFN-beta-1a or subcutaneous (SC) GA two Phase 1, open-label, single-center, randomized, crossover studies each enrolled 26 healthy volunteers. Dosing sequences comprised two dosing periods separated by 7 days. Treatment consisted of DMF 240 mg tid for 2 or 3 days alone (GA and IFN-beta-1a respectively) or given with a single IM IFN-beta-1a 30-µg or SC GA 20-mg injection administered on Day 2. Pharmacokinetic (PK) parameters, vital signs, ECG, and adverse events (AEs) were assessed.

Twenty-five subjects completed the DMF + GA study and 24 subjects completed the DMF + IFN-beta-1a study. DMF metabolite (monomethyl fumarate [MMF]) concentrations in all treatment groups were comparable, suggesting no clinically significant effect of IM IFN-beta-1a or GA on DMF disposition. The most common adverse events were flushing with DMF alone (both studies) and DMF + GA, and flu-like symptoms with DMF + IM IFN-beta-1a. There were no serious AEs or deaths. One subject with moderate increase in liver enzymes and neutropenia following DMF + IM IFN-beta-1a treatment withdrew from the study. In the DMF + GA study, one subject discontinued after receiving DMF alone due to a mild erythematous facial nodule and 1 subject discontinued for mild nausea after receiving DMF + GA. A mild increase in temperature and pulse was observed only following IM IFN-beta-1a administration; no subject withdrew for these reasons. Hematological shifts were observed in subjects receiving DMF alone and DMF + GA (mild neutropenia and anemia); no subject withdrew for these reasons. No clinically significant abnormalities on physical examination or ECG were observed. Hence, no new safety signals were raised in this study in healthy volunteers given DMF alone or in combination with IM IFN-beta-1a or GA (Table 2).

These results also show that the PK profile of DMF was not altered by coadministration with IM IFN-beta-1a or GA, indicating no drug interaction.

**Table 2: Summary of the most common adverse events (AE)**

| | IFN-beta-1a | | GA | |
|---|---|---|---|---|
| AE (%) | DMF Alone (n=24) | DMF + IFN-beta-1a (n=26) | DMF Alone (n=25). | DMF + GA (n=25) |
| Any AE | 71 | 100 | 84 | 92 |
| Flushing | 50 | 81 | 76 | 85 |
| Influenza-like illness | 0 | 85 | 0 | 0 |
| Paresthesia | 21 | 23 | 32 | 19 |
| Pruritus | 4 | 8 | 28 | 19 |
| Headache | 13 | 8 | 12 | 15 |
| Diarrhea | 0 | 0 | 8 | 15 |
| Nausea | 4 | 4 | 8 | 12 |

### Example 7:

### Exemplary Formulation for DMF

In this example, DMF is formulated in capsules containing enterically coated microtablets. The coating of the microtablets is composed of different layers. The first layer is a methacrylic acid - methyl methacrylate copolymer/isopropyl alcohol solution which isolates the tablet cores from potential hydrolysis from the next applied water suspensions. Enteric coating of the tablet is then conferred by an aqueous methacrylic acid-ethyl acrylate copolymer suspension. The complete components and quantitative composition of the capsules are given in Table 3.

**Table 3: Exemplary formulation for DMF**

| **Ingredients** | **Amount/capsule** | **Function** |
|---|---|---|
| | | |

| **Core Microtablets** | | |
|---|---|---|
| *Active ingredients:* | | |
| Dimethyl Fumarate* | 120.00 mg | active ingredient |
| *Excipients:* | | |
| Croscarmellose sodium | 15.00 mg | disintegrant |
| Microcrystalline Cellulose | 131.60 mg | filler |
| Magnesium stearate | 5.00mg | lubricant |
| Talcum | 19.80mg | glidant |
| Silica colloidal anhydrous | 2.60mg | glidant |
| *Mass core microtablets* | 294.00mg | |
| | | |

| **Coating Microtablets** | | |
|---|---|---|
| *Excipients:* | | |
| Triethyl Citrate** | 7.60mg | plasticizer |
| Methacrylic Acid-Methyl | | |
| Methacrylate Copolymer (1:1) | 5.50mg | |
| as | | film coating agent |
| Methacrylic Acid-Methyl | | |
| Methacrylate Copolymer (1:1) | | |
| solution 12.5%** | (44.00 mg) | |
| Simeticone (corresponding to Simeticone Ph Eur) | 0.17 mg | |
| as | | anti-foam agent |
| Simeticone Emulsion USP** | (0.53 mg) | |
| Talcum micronised** | 13.74 mg | lubricant |
| Methacrylic acid - Ethyl Acrylate Copolymer (1:1) | 33.00 mg | film coating agent |
| as | | |
| Methacrylic acid - Ethyl | | |
| Acrylate Copolymer (1:1) | | |
| dispersion 30% ** | (110.00 mg) | |
| *Mass enteric coated microtablets* | 354.01 mg | |
| *Mass of gelatin capsule* | 96.00mg | |
| *Mass offilled capsule* | 450.01 mg | |

The manufacturing process and process controls include the following:
A) Active and non-active ingredients are weighed and each starting material is identified with an appropriate labelling (denomination, batch number, quantity).
B) Blending: A powder mixture containing the active ingredient dimethyl fumarate and all excipients of the core microtablets is prepared.
C) Tabletting: A rotative press is equipped with multiple-punches tools, a deduster and the powder mixture is tabletted according to the given specifications.
D) Film Coating: In accordance with commonly used film coating methods the microtablet cores are isolated by spraying an isolation solution using a film coating equipment. The isolated cores are sprayed with an enteric coating suspension in the film coating pan. The gastro-resistance of microtablets and the active ingredient content are controlled.
E) Capsule Filling: Based on microtablets active ingredient the capsules are filled with an amount corresponding to 120 mg of active ingredient per capsule. The capsule filling weight and capsule length are controlled.
F) Packaging: The capsules are packaged on a blistering machine in thermoformed PVC/PE/PVdC - Aluminium blisters.

### Example 8:

### Effects of Combination Therapies Involving DMF/Glatiramer Acetate as well as DMF/IFN-beta in Mice

The effects of combination therapy with suboptimal doses of GLAT (50 mg s.c. as co-immunization with MOG) or murine interferon beta (10.000U s.c. three times weekly) and DMF (15 mg/kg BW) on the course of MOG-EAE (n= 6 per group) were studied. In the acute disease phase (day 26 or day 18 p.i., respectively), parameters of inflammation as well as CNS tissue integrity were analyzed. To this end, 12 week wild-type or C57BL/6 mice were actively immunized with 200 mg of MOG peptide 35-55 and pertussis toxin. On day 18 or 26 p.i., histochemistry was perfomed to identify T cells (CD3 immunohistochemistry), macrophages/microglia (Mac-3), demyelination (Luxol Fast Blue staining), and axonal loss (Bielschowsky silver impregnation). Histological changes were quantified by a blinded observer counting six visual fields on spinal cord cross sections comprising cervical, thoracic and lumbar spinal cord. Statistical evaluations were performed by Kruskal-Wallis test or t-test. ** p < 0.01; *** p < 0.001.

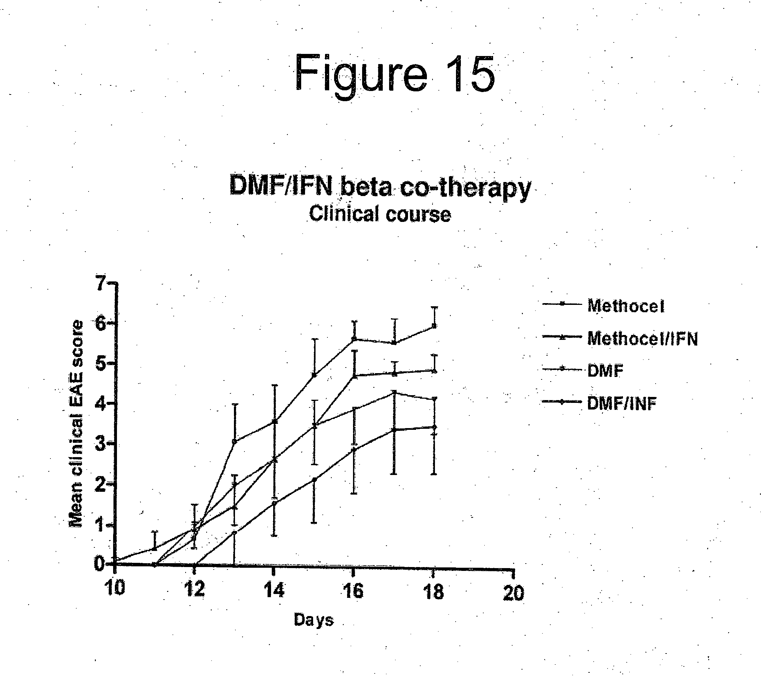

Results: Treatment with DMF and GA or DMF and IFN-beta both resulted in beneficial effects on the course of acute MOG-EAE (Figure 10A and Figure 15).

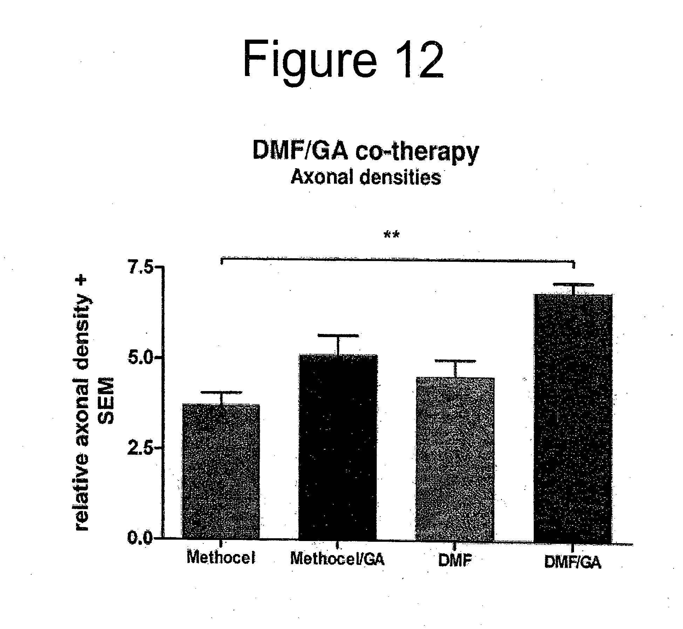

The combination of DMF and GA led to a significant axon preservation on day 26 p.i. (Figure 12) and also in the chronic phase of the disease (data not shown).

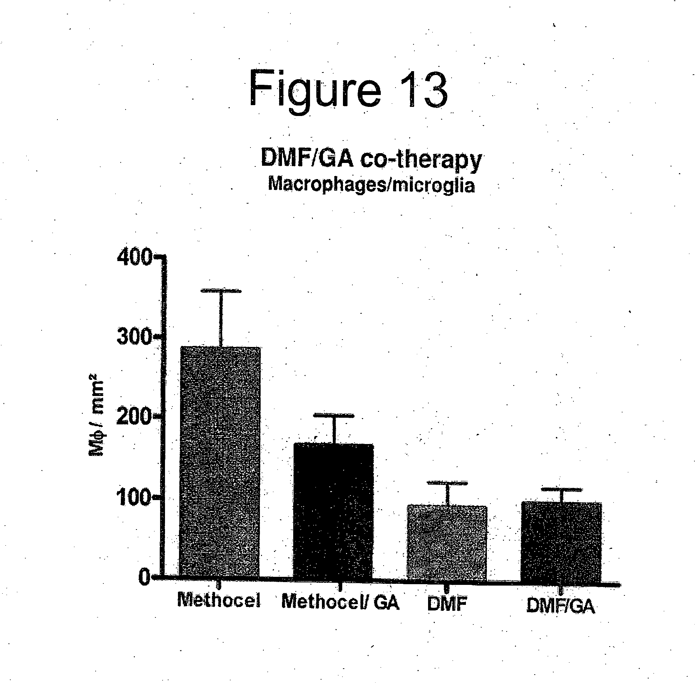

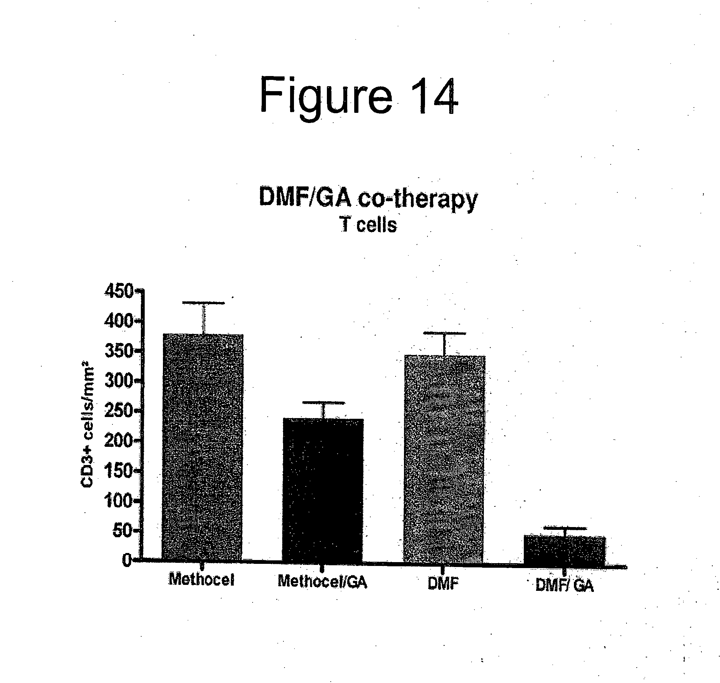

While DMF alone efficiently reduces numbers of macrophages/microglia, combination of DMF and GA also leads to significant lower numbers of T cells in EAE lesions (Figure 13 and Figure 14).

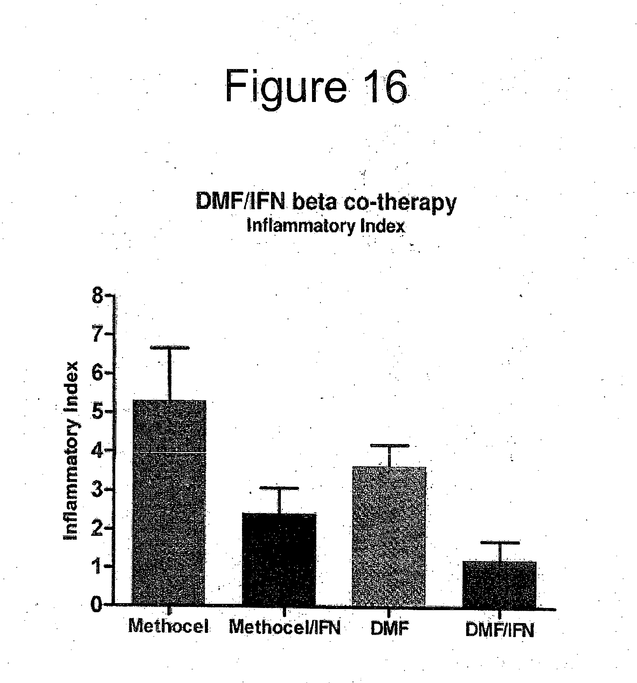

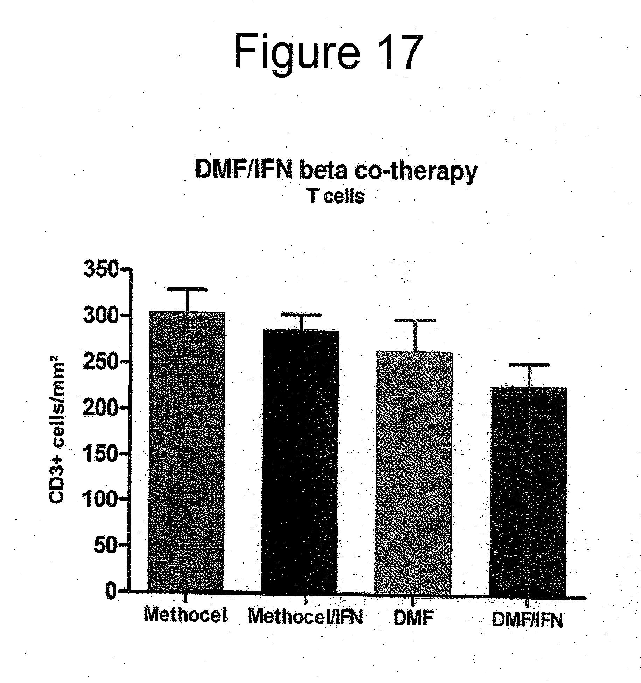

In contrast DMF/IFN-beta co-therapy mainly reduces overall numbers of infiltrates without altering the amount of infiltrating cells once a lesions is established (Figure 16 and Figure 17).

In all experiments, combination therapy was well tolerated.

Conclusion: DMF may synergize with other immunomodulatory agents in the treatment of CNS autoimmunity. These data provide a rationale for testing combination therapies including DMF in MS clinical trials.

Having now fully described this invention, it will be understood by those of ordinary skill in the art that the same can be performed within a wide and equivalent range of conditions, formulations and other parameters without affecting the scope of the invention or any embodiment thereof.

Other embodiments of the invention will be apparent to those skilled in the art from consideration of the specification and practice of the invention disclosed herein. It is intended that the specification and examples be considered as exemplary only.

## Claims

1. A combination therapy consisting of (a) dimethyl fumarate (DMF) and/or monomethyl fumarate (MMF) and (b) glatiramer acetate, for use in treating a subject having multiple sclerosis (MS).

2. The combination therapy for use according to claim 1, wherein 20 mg/day of glatiramer acetate is to be administered to the subject.

3. The combination therapy for use according to claim 1, wherein less than 20 mg/day of glatiramer acetate is to be administered to the subject.

4. The combination therapy for use according to any one of the preceding claims, wherein the DMF and/or MMF and the glatiramer acetate is for use in (i) reducing a relapse rate in the subject; or (ii) reducing demyelination and/or axonal death in the subject.

5. The combination therapy for use according to any one of the preceding claims, wherein the DMF and/or MMF and the glatiramer acetate are formulated into a single dosage form.

6. The combination therapy for use according to any one of claims 1 to 4, wherein the DMF and/or MMF and the glatiramer acetate are formulated into separate dosage forms for administration separately or together.

7. A combination therapy consisting of (a) DMF and/or MMF, and (b) interferon-beta, for use in treating a subject having MS.

8. The combination therapy for use according to claim 7, wherein the interferon-beta is selected from interferon-beta 1a and interferon-beta 1b.

9. The combination therapy for use according to claim 8, wherein the interferon-beta is interferon-beta 1 a.

10. The combination therapy for use according to claim 9, wherein the interferon-beta 1a is to be administered at a dose of 5 mcg to 80 mcg at one time.

11. The combination therapy for use according to claim 10, wherein the interferon-beta 1a is to be administered once a week.

12. The combination therapy for use according to any one of the preceding claims, wherein the MS is selected from relapsing remitting MS, secondary progressive MS, primary progressive MS, progressive relapsing MS, and clinically isolated syndrome.

13. The combination therapy for use according to any one of the preceding claims, wherein the compound selected from DMF, MMF, and combinations thereof is DMF.

14. The combination therapy for use according to claim 13, wherein 240 mg of DMF is to be administered to the subject at one time.

15. The combination therapy for use according to claim 14, wherein the DMF is to be administered to the subject two times per day (BID) equivalent to a total daily dose of 480 mg per day.

## Patentansprüche

1. Kombinationstherapie bestehend aus (a) Dimethylfumarat (DMF) und/oder Monomethylfumarat (MMF) und (b) Glatirameracetat zur Verwendung bei der Behandlung eines Subjekts, das Multiple Sklerose (MS) aufweist.

2. Kombinationstherapie zur Verwendung nach Anspruch 1, wobei dem Subjekt 20 mg/Tag Glatirameracetat verabreicht werden.

3. Kombinationstherapie zur Verwendung nach Anspruch 1, wobei dem Subjekt weniger als 20 mg/Tag Glatirameracetat verabreicht werden.

4. Kombinationstherapie zur Verwendung nach einem der vorherigen Ansprüche, wobei das DMF und/oder MMF und das Glatirameracetat zur Verwendung sind zur (i) Reduzierung einer Relapsrate bei dem Subjekt oder zur (ii) Reduzierung der Demyelinisierung und/oder des Axonal-Sterbens bei dem Subjekt.

5. Kombinationstherapie zur Verwendung nach einem der vorherigen Ansprüche, wobei das DMF und/oder MMF und das Glatirameracetat in einer Einzeldarreichungsform formuliert sind.

6. Kombinationstherapie zur Verwendung nach einem der Ansprüche 1 bis 4, wobei das DMF und/oder MMF und das Glatirameracetat in unterschiedlichen Darreichungsformen formuliert sind zur getrennten oder gemeinsamen Verabreichung.

7. Kombinationstherapie bestehend aus (a) DMF und/oder MMF und (b) Beta-Interferon zur Verwendung bei der Behandlung eines Subjekts, das MS aufweist.

8. Kombinationstherapie zur Verwendung nach Anspruch 7, wobei das Beta-Interferon ausgewählt ist aus Beta-Interferon 1a und Beta-Interferon 1b.

9. Kombinationstherapie zur Verwendung nach Anspruch 8, wobei das Beta-Interferon Beta-Interferon 1a ist.

10. Kombinationstherapie zur Verwendung nach Anspruch 9, wobei das Beta-Interferon 1a in einer Dosierung von 5 mcg bis 80 mcg einmal verabreicht wird.

11. Kombinationstherapie zur Verwendung nach Anspruch 10, wobei das Beta-Interferon 1a einmal wöchentlich verabreicht wird.

12. Kombinationstherapie zur Verwendung nach einem der vorherigen Ansprüche, wobei die MS ausgewählt ist aus relapsierend-remittierender MS, sekundärerprogredienter MS, primärer-progredienter MS, progredienter-relapsierender MS und klinisch isoliertem Syndrom.

13. Kombinationstherapie zur Verwendung nach einem der vorherigen Ansprüche, wobei die aus DMF, MMF und Kombinationen davon ausgewählte Verbindung DMF ist.

14. Kombinationstherapie zur Verwendung nach Anspruch 13, wobei dem Subjekt einmal 240 mg DMF verabreicht werden.

15. Kombinationstherapie nach Anspruch 14, wobei das DMF dem Subjekt zweimal am Tag verabreicht wird (BID), was einer täglichen Gesamtdosis von 480 mg pro Tag entspricht.

## Revendications

1. Polythérapie combinant (a) du diméthyl fumarate (DMF) et/ou du monométhyl fumarate (MMF) et (b) de l'acétate de glatiramère pour son utilisation dans le traitement d'un sujet atteint de sclérose en plaques (SP).

2. Polythérapie pour son utilisation selon la revendication 1, dans laquelle 20 mg/jour d'acétate de glatiramère doivent être administrés au sujet.

3. Polythérapie pour son utilisation selon la revendication 1, dans laquelle moins de 20 mg/jour d'acétate de glatiramère doivent être administrés au sujet.

4. Polythérapie pour son utilisation selon l'une quelconque des revendications précédentes, dans laquelle le DMF et/ou le MMF et l'acétate de glatiramère sont destinés à être utilisés pour (i) réduire le taux de récidive chez le sujet ; ou (ii) réduire la démyélinisation et/ou la mort axonale chez le sujet.

5. Polythérapie pour son utilisation selon l'une quelconque des revendications précédentes, dans laquelle le DMF et/ou le MMF et l'acétate de glatiramère sont formulés en une seule forme galénique.

6. Polythérapie pour son utilisation selon l'une quelconque des revendications 1 à 4, dans laquelle le DMF et/ou le MMF et l'acétate de glatiramère sont formulés en formes galéniques distinctes pour une administration séparée ou simultanée.

7. Polythérapie combinant (a) du DMF et/ou du MMF et (b) l'interféron bêta pour son utilisation dans le traitement d'un sujet atteint de SP.

8. Polythérapie pour son utilisation selon la revendication 7, dans laquelle l'interféron bêta est sélectionné parmi l'interféron bêta 1a et l'interféron bêta 1b.

9. Polythérapie pour son utilisation selon la revendication 8, dans laquelle l'interféron bêta est l'interféron bêta 1a.

10. Polythérapie pour son utilisation selon la revendication 9, dans laquelle l'interféron bêta 1a doit être administré à une dose de 5 µg à 80 µg par prise.

11. Polythérapie pour son utilisation selon la revendication 10, dans laquelle l'interféron bêta 1a doit être administré une fois par semaine.

12. Polythérapie pour son utilisation selon l'une quelconque des revendications précédentes, dans laquelle la SP est sélectionnée parmi la SP récurrente-rémittente, la SP progressive secondaire, la SP progressive primaire, la SP progressive récurrente, et un syndrome cliniquement isolé.

13. Polythérapie pour son utilisation selon l'une quelconque des revendications précédentes, dans laquelle le composé sélectionné parmi le DMF, le MMF, et les combinaisons de ceux-ci est le DMF.

14. Polythérapie pour son utilisation selon la revendication 13, dans laquelle 240 mg de DMF doivent être administrés au sujet en une prise.

15. Polythérapie pour son utilisation selon la revendication 14, dans laquelle le DMF doit être administré au sujet deux fois par jour, équivalant à une dose quotidienne totale de 480 mg par jour.
